# DEMANDE DE BREVET EUROPEEN

(11) **EP 1 568 687 A1**
(43) Date de publication de la demande: **31.08.2005**
(21) Numéro de dépôt: 05290420.8
(22) Date de dépôt: 24.02.2005
(51) Int. Cl.: C07C 311/18, C07C 311/32, C07C 317/28, C07C 323/52, C07C 381/02, C07D 307/14, A61K 7/13

(54) **Para-phénylènediamines secondaires soufrées composition tinctoriale comprenant de telles para-phénylènediamines procédé mettant en oeuvre cette composition et utilisation**

(30) Priorité: 27.02.2004 FR 0402016
(71) Demandeur: L'OREAL, 75008 Paris (FR)
(72) Inventeur: Sabelle, Stéphane, 75005 Paris (FR)
(74) Mandataire: Dossmann, Gérard

(57) **Abrégé**

La présente invention a pour objet une nouvelle famille de para-phénylènediamines secondaires soufrées, leur préparation, leur utilisation en coloration capillaire, une composition tinctoriale pour la teinture des fibres kératiniques, en particulier des fibres kératiniques humaines, telles que les cheveux, comprenant dans un milieu de teinture approprié au moins une para-phénylènediamine secondaire soufrée.

L'invention a aussi pour objet le procédé mettant en oeuvre cette composition, son utilisation et le dispositif à plusieurs compartiments ou « kit » de teinture.

## Description

La présente demande concerne une nouvelle famille de para-phénylènediamines secondaires soufrés, leur préparation et leur utilisation en coloration d'oxydation capillaire.

Il est connu de teindre les fibres kératiniques et en particulier les cheveux humains avec des compositions tinctoriales contenant des précurseurs de colorant d'oxydation, appelés généralement bases d'oxydation, tels que des ortho ou para-phénylènediamines, des ortho ou para-aminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les méta-diamino benzènes aromatiques, les méta-aminophénols, les méta-diphénols et certains composés hétérocycliques tels que des composés indoliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (i.e. abîmée) entre sa pointe et sa racine.

De manière surprenante et avantageuse, la Demanderesse vient de découvrir une nouvelle famille de para-phénylènediamines secondaires soufrées, capables de conduire à des colorations aux nuances variées, puissantes, esthétiques, peu sélectives et résistant bien aux diverses agressions que peuvent subir les fibres. La présente demande porte également sur un procédé de préparation de ces para-phénylènediamines secondaires soufrées ainsi que sur leur utilisation en coloration d'oxydation capillaire.

L'invention a également pour objet de nouvelles compositions pour la teinture des fibres kératiniques, en particulier des fibres kératiniques humaines telles que les cheveux, comprenant au moins une para-phénylènediamines secondaires soufrées.

La composition de la présente invention permet en particulier d'obtenir une coloration de fibres kératiniques très puissante, peu sélective et tenace, en particulier à la lumière, tout en évitant la dégradation de ces fibres. En outre, ces compositions présentent un bon profil toxicologique.

L'invention a aussi pour objet le procédé de teinture mettant en oeuvre cette composition selon la présente invention pour la teinture des fibres kératiniques, en particulier des fibres kératiniques humaines telles que les cheveux, l'utilisation de cette composition, ainsi que le dispositif à plusieurs compartiments ou "kit" de teinture.

D'autres caractéristiques, aspects, objets et avantages de la présente invention apparaîtront encore plus clairement à la lecture de la description et des exemples qui suivent.

Dans le cadre de la présente invention, on entend par alkyle, un radical linéaire ou ramifié en C₁-C₁₅, par exemple méthyle, éthyle, n-propyle, iso-propyle, butyle, etc. Un radical alcoxy est un radical alk-O, le radical alkyle ayant la définition donnée ci dessus, par exemple méthyloxy, éthyloxy. Halogène désigne de préférence Cl, Br, I, F.
Les nouvelles para-phénylènediamines secondaires soufrées selon la présente invention sont des composés de formule générale (I) : 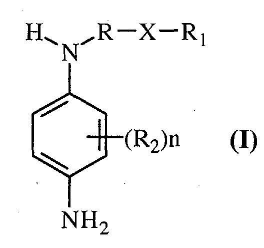 dans laquelle :
le groupement X représente un radical SO₂ ou un atome de soufre,
si X représente un radical SO₂, le groupement R représente un radical alkylène en C₂-C₁₀ linéaire ou ramifié, saturé ou insaturé, non-substitué ou substitué par un atome d'halogène ou par un ou plusieurs groupements hydroxy, alcoxy, amino, mono- ou di-alkylamino, alkylcarbonyle, carboxy, amido, alcoxycarbonyle ou mono- ou di-alkylamino carbonyle ; un ou plusieurs atomes de carbone de ce radical alkylène pouvant être remplacés par un ou plusieurs hétéroatomes choisi parmi l'oxygène, l'azote et le soufre, ou par une ou plusieurs fonctions carbonyles,
si X représente un atome de soufre, le groupement R représente un radical alkylène en C₁-C₁₀ ramifié, saturé ou insaturé, non-substitué ou substitué par un atome d'halogène ou par un ou plusieurs groupements hydroxy, alcoxy, amino, mono- ou di-alkylamino, alkylcarbonyle, carboxy, amido, alcoxycarbonyle ou mono- ou di-alkylamino carbonyle ; un ou plusieurs atomes de carbones de ce radical alkylène pouvant être remplacés par un ou plusieurs hétéroatomes choisi parmi l'oxygène, l'azote et le soufre, ou par une ou plusieurs fonctions carbonyles,
le groupement R₁ représente un radical amino, un radical aryle ou un radical alkyle en C₁-C₁₀ linéaire ou ramifié, saturé ou insaturé, non-substitué ou substitué par un atome d'halogène ou par un ou plusieurs groupements hydroxy, alcoxy, amino, mono- ou di-alkylamino, alkylcarbonyle, carboxy, amido, alcoxycarbonyle, mono- ou di-alkylamino carbonyle ou un hétérocycle à 5 ou 6 atomes saturé ou insaturé ; un ou plusieurs atomes de carbone de ce radical alkyle pouvant être remplacés par un ou plusieurs hétéroatomes choisi parmi l'oxygène, l'azote et le soufre, ou par une ou plusieurs fonctions carbonyles,
le groupement R₂ représente un atome d'hydrogène ou d'halogène, un radical choisi parmi alkyle, alcoxy, alcoxy alkyle, hydroxy alcoxy ou mono- ou poly-hydroxy alkyle,
n représente un entier variant de 1 à 4,
ou ses sels d'addition
à l'exception des composés suivants :

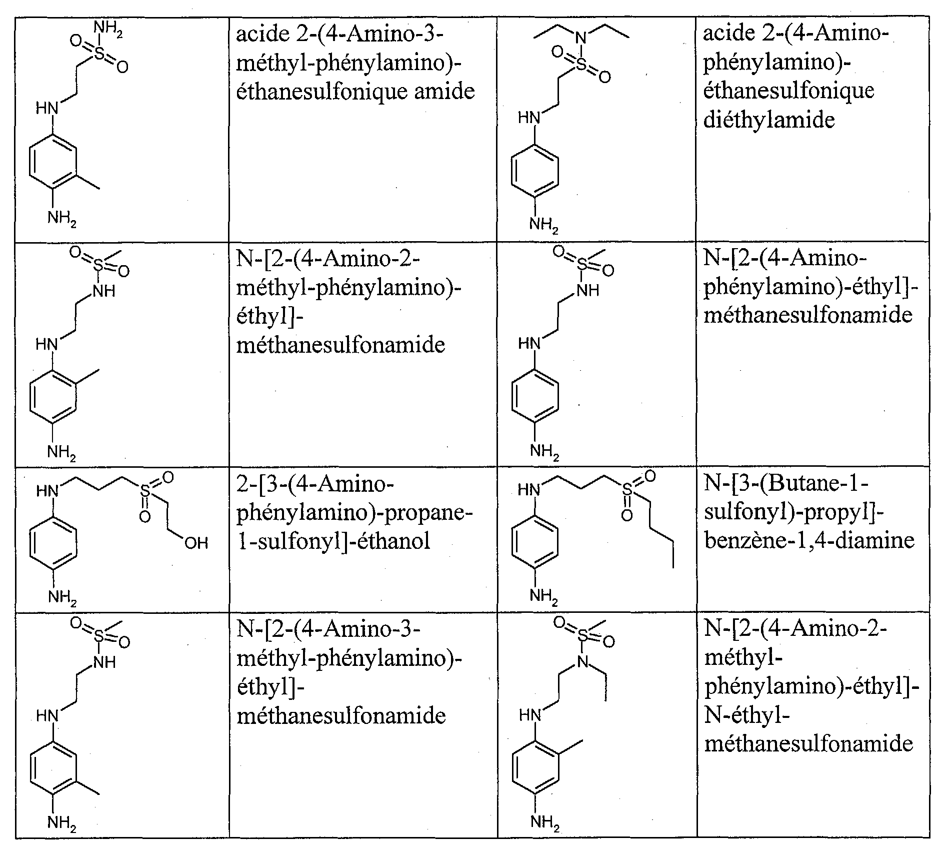

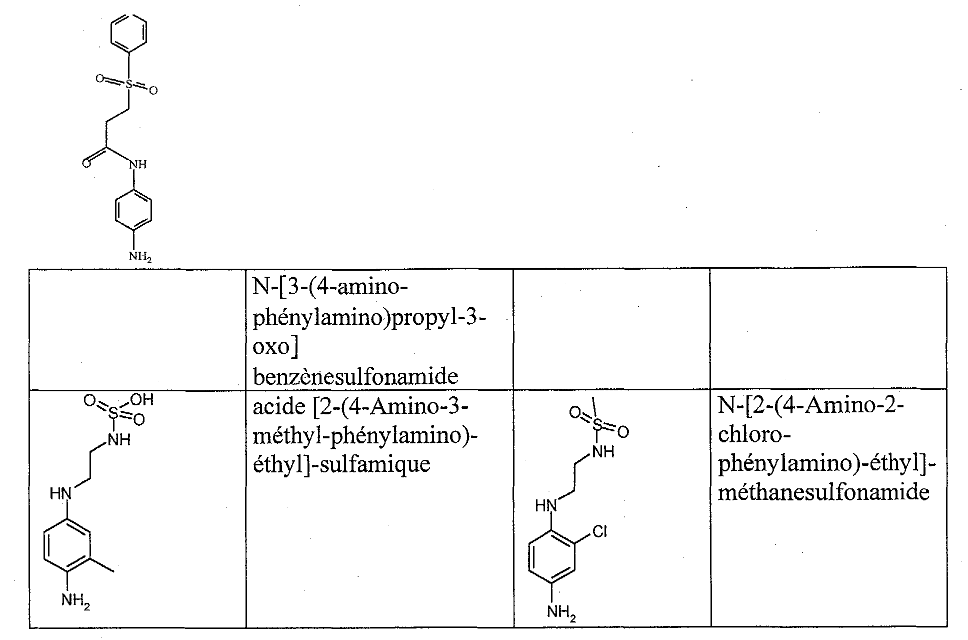

Par un radical aryle, on entend au sens de la présente invention, un radical aryle non-substitué.

De manière préférée, le groupement R de la formule (I) représente un alkylène linéaire ou ramifié en C₂-C₅, pouvant être substitué par un radical choisi parmi -OH, -COOCH₃ et -COOCH₂CH_{3,} un ou plusieurs atomes de carbone de ce radical alkylène pouvant être remplacés par un ou plusieurs atomes d'oxygène, d'azote ou de soufre.

De manière préférée, le groupement R₁ de la formule (I) représente un groupement alkyle, aryle, arylalkyle, hydroxyalkyle, amine secondaire ou tertiaire.

De manière préférée, le groupement R₂ de la formule (I) représente un atome d'hydrogène ou un groupement méthyle. Les composés plus particulièrement préférés sont :

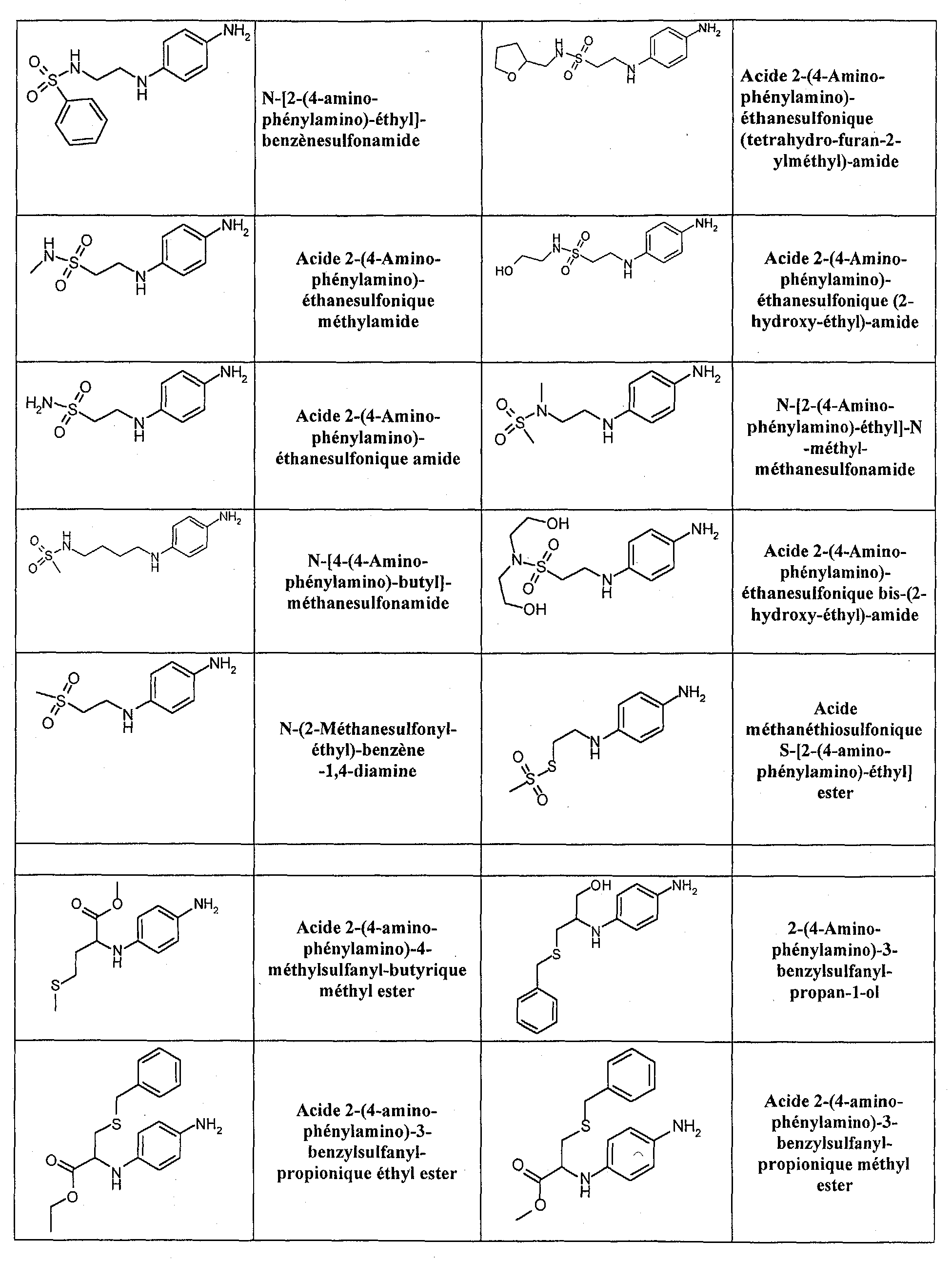

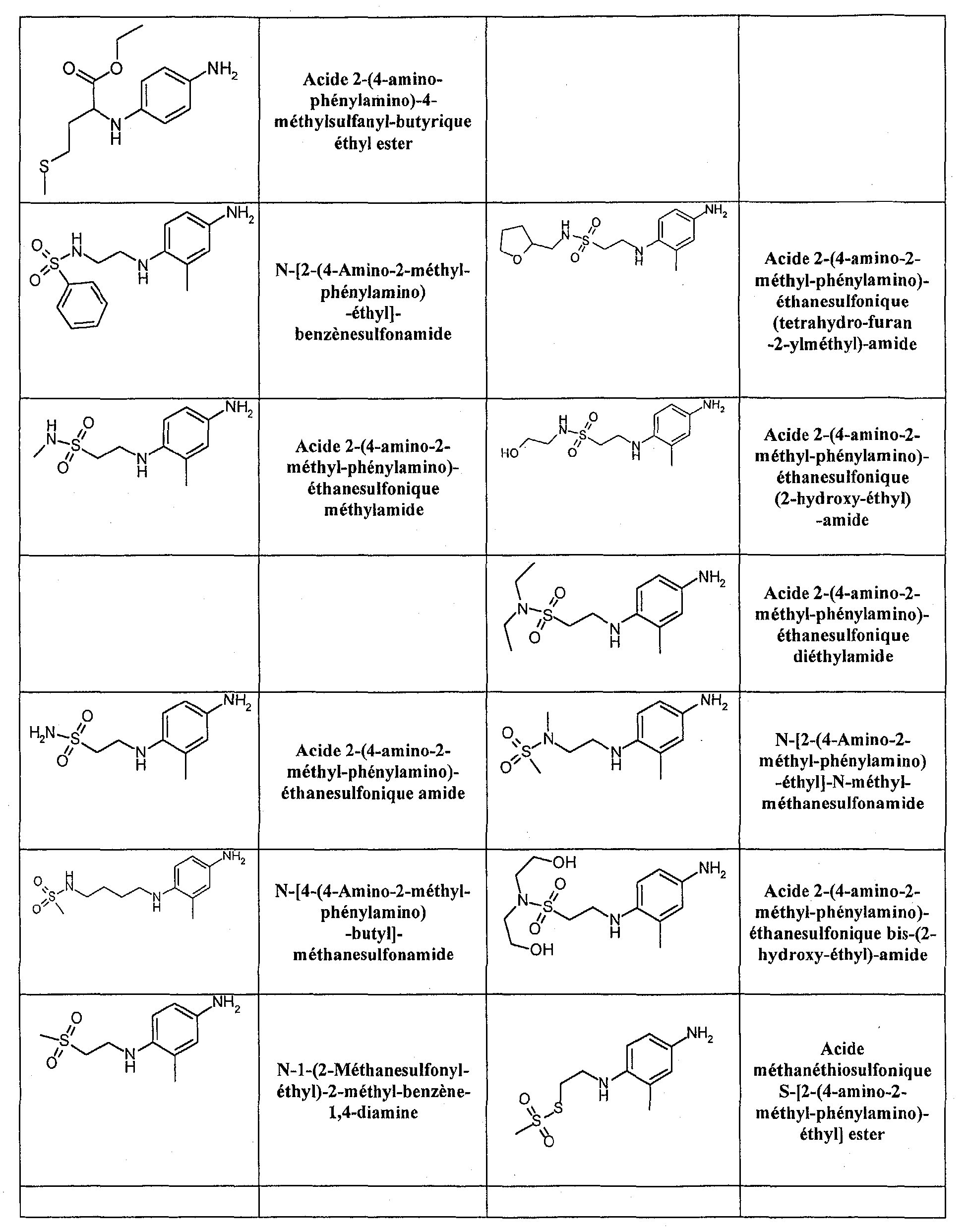

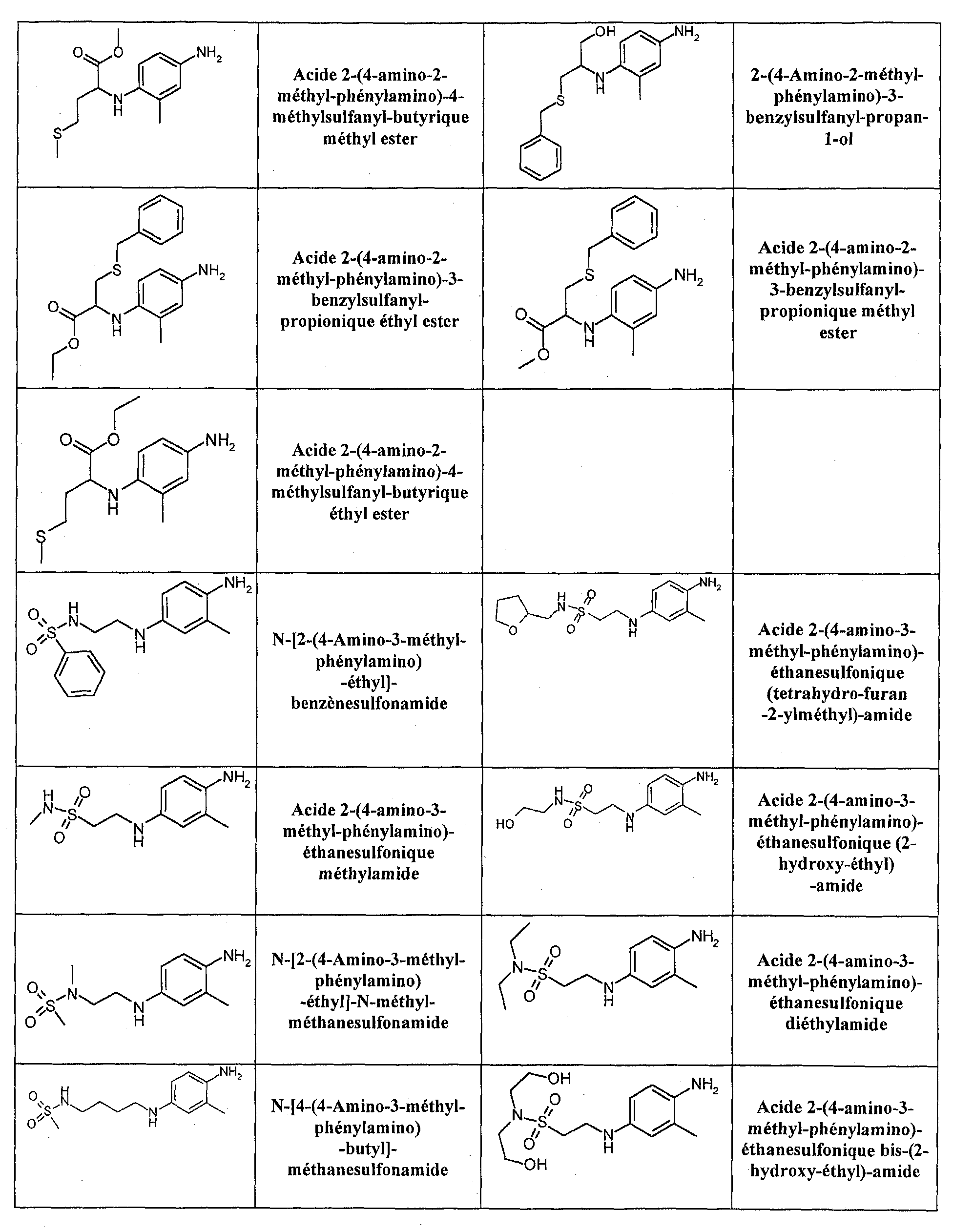

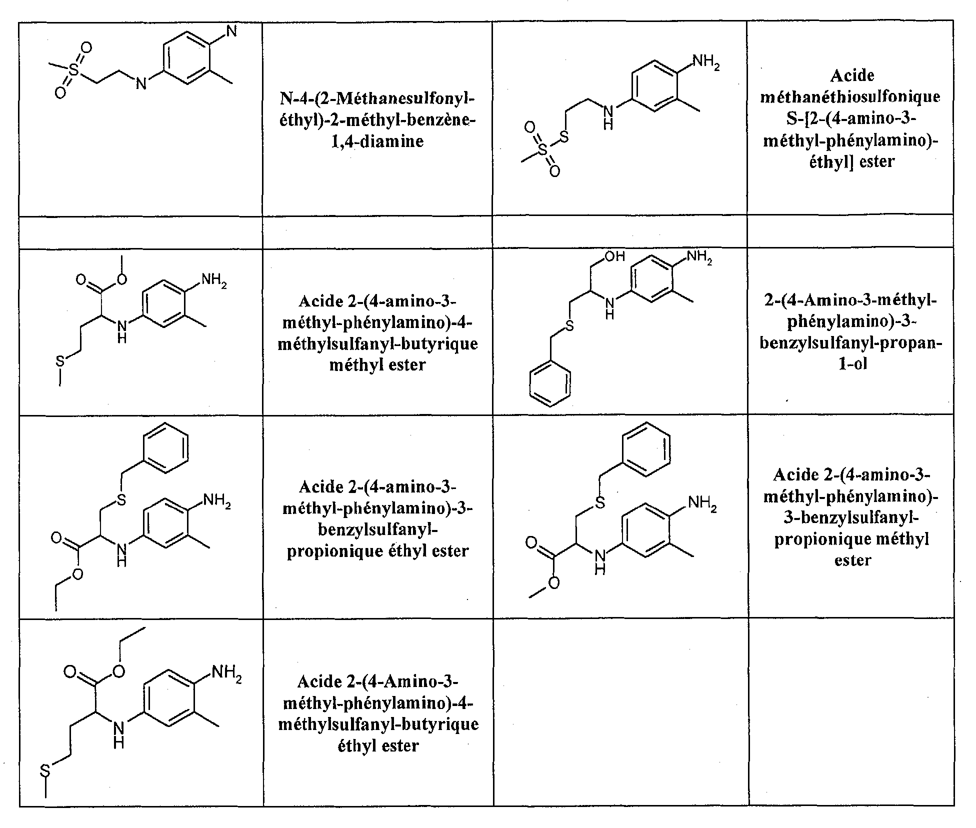

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates.

Les composés de formule (I) selon la présente demande peuvent être préparés de façon générale suivant un procédé comprenant les étapes suivantes :
- substitution nucléophile de l'halogène en position para- dans le dérivé para-halogénonitrobenzène par une amine primaire de formule R₁XRNH₂ en présence d'une base (R₁, X et R étant tels que définis précédemment), et
- réduction de la fonction nitro du composé obtenu à l'étape précédente en fonction amine pour obtenir le composé de formule (I).

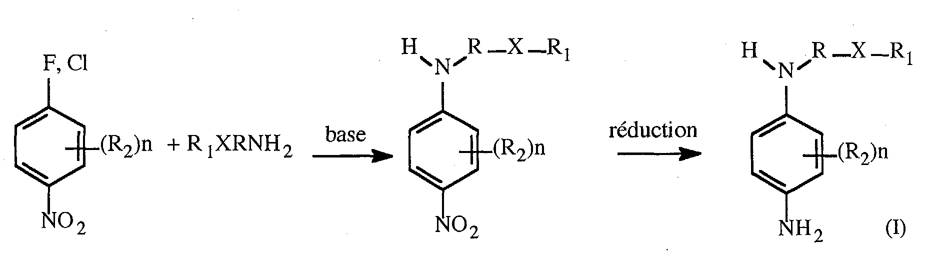

La première étape de la synthèse est décrite dans les revues Synthesis 1990 (12), 1147-1148 et Synth. Commun. 1990, 20 (22), 3537-3545. La deuxième étape est une étape de réduction classique, par exemple en effectuant une réaction d'hydrogénation par catalyse hétérogène en présence de Pd/C, Pd(II)/C, Ni de Raney ou encore en effectuant une réaction de réduction par un métal, par exemple par du zinc, fer, étain... (Advanced Organic Chemistry, 4^{th} edition, 1992, J. MARCH, WILEY Interscience ; Reduction in Organic Chemistry, M. Hudlicky, 1983, Ellis Honwood series Chemical Science).

La présente demande concerne aussi les composés nitrés de formule (II) et les procédés de préparation des composés para-phénylènediamines secondaires de formule (I), dans lesquels on effectue une étape de réduction du composé nitré correspondant, le composé nitré correspondant étant le composé de formule (I) dans lequel le groupe amino en para du groupe NHXR₁ est remplacé par un groupe nitro. 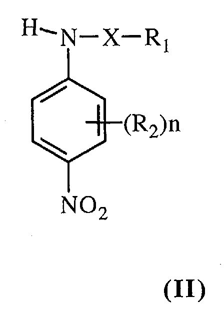 La présente demande porte également sur l'utilisation du composé de formule générale (I) pour la coloration d'oxydation capillaire : 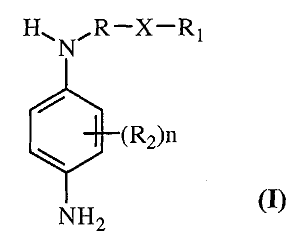 dans laquelle:
le groupement X représente un radical SO₂ ou un atome de soufre,
si X représente un radical SO₂, le groupement R représente un radical alkylène en C₂-C₁₀ linéaire ou ramifié, saturé ou insaturé, non-substitué ou substitué par un atome d'halogène ou par un ou plusieurs groupements hydroxy, alcoxy, amino, mono- ou di-alkylamino, alkylcarbonyle, carboxy, amido, alcoxycarbonyle ou mono- ou di-alkylamino carbonyle ; un ou plusieurs atomes de carbone de ce radical alkylène pouvant être remplacés par un ou plusieurs hétéroatomes choisi parmi l'oxygène, l'azote et le soufre, ou par une ou plusieurs fonctions carbonyles,
si X représente un atome de soufre, le groupement R représente un radical alkylène en C₁-C₁₀ ramifié, saturé ou insaturé, non-substitué ou substitué par un atome d'halogène ou par un ou plusieurs groupements hydroxy, alcoxy, amino, mono- ou di-alkylamino, alkylcarbonyle, carboxy, amido, alcoxycarbonyle ou mono- ou di-alkylamino carbonyle ; un ou plusieurs atomes de carbones de ce radical alkylène pouvant être remplacés par un ou plusieurs hétéroatomes choisi parmi l'oxygène, l'azote et le soufre, ou par une ou plusieurs fonctions carbonyles,
le groupement R₁ représente un radical amino, un radical -OY, dans lequel Y représente un atome d'hydrogène ou un métal, un radical aryle ou un radical alkyle en C₁-C₁₀ linéaire ou ramifié, saturé ou insaturé, non-substitué ou substitué par un atome d'halogène ou par un ou plusieurs groupements hydroxy, alcoxy, amino, mono- ou di-alkylamino, alkylcarbonyle, carboxy, amido, alcoxycarbonyle, mono- ou di-alkylamino carbonyle ou un hétérocycle à 5 ou 6 atomes saturé ou insaturé ; un ou plusieurs atomes de carbone de ce radical alkyle pouvant être remplacés par un ou plusieurs hétéroatomes choisi parmi l'oxygène, l'azote et le soufre, ou par une ou plusieurs fonctions carbonyles,
le groupement R₂ représente un atome d'hydrogène ou d'halogène, un radical choisi parmi alkyle, alcoxy, alcoxy alkyle, hydroxy alcoxy ou mono- ou poly-hydroxy alkyle,
n représente un entier variant de 1 à 4,
ou ses sels d'addition
à l'exception des composés suivants :

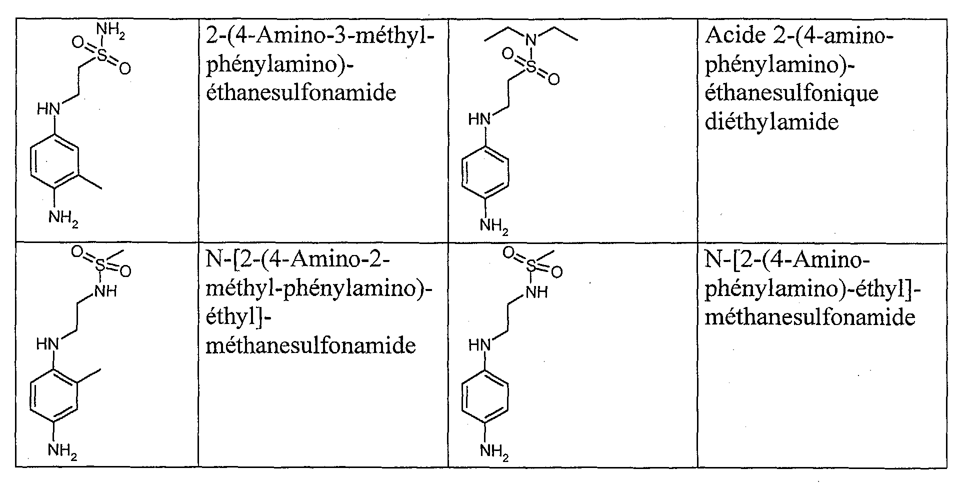

La présente demande concerne également une composition cosmétique pour la teinture des fibres, de préférence les fibres kératiniques humaines, telles que les cheveux, comprenant, dans un milieu approprié pour la teinture, au moins un composé de formule générale (I) : 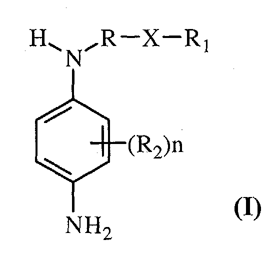 dans laquelle :
le groupement X représente un radical SO₂ ou un atome de soufre,
si X représente un radical SO₂, le groupement R représente un radical alkylène en C₂-C₁₀ linéaire ou ramifié, saturé ou insaturé, non-substitué ou substitué par un atome d'halogène ou par un ou plusieurs groupements hydroxy, alcoxy, amino, mono- ou di-alkylamino, alkylcarbonyle, carboxy, amido, alcoxycarbonyle ou mono- ou di-alkylamino carbonyle ; un ou plusieurs atomes de carbone de ce radical alkylène pouvant être remplacés par un ou plusieurs hétéroatomes choisi parmi l'oxygène, l'azote et le soufre, ou par une ou plusieurs fonctions carbonyles,
si X représente un atome de soufre, le groupement R représente un radical alkylène en C₁-C₁₀ ramifié, saturé ou insaturé, non-substitué ou substitué par un atome d'halogène ou par un ou plusieurs groupements hydroxy, alcoxy, amino, mono- ou di-alkylamino, alkylcarbonyle, carboxy, amido, alcoxycarbonyle ou mono- ou di-alkylamino carbonyle ; un ou plusieurs atomes de carbones de ce radical alkylène pouvant être remplacés par un ou plusieurs hétéroatomes choisi parmi l'oxygène, l'azote et le soufre, ou par une ou plusieurs fonctions carbonyles,
le groupement R₁ représente un radical amino, un radical -OY, dans lequel Y représente un atome d'hydrogène ou un métal, un radical aryle ou un radical alkyle en C₁-C₁₀ linéaire ou ramifié, saturé ou insaturé, non-substitué ou substitué par un atome d'halogène ou par un ou plusieurs groupements hydroxy, alcoxy, amino, mono- ou di-alkylamino, alkylcarbonyle, carboxy, amido, alcoxycarbonyle, mono- ou di-alkylamino carbonyle ou un hétérocycle à 5 ou 6 atomes saturé ou insaturé ; un ou plusieurs atomes de carbone de ce radical alkyle pouvant être remplacés par un ou plusieurs hétéroatomes choisi parmi l'oxygène, l'azote et le soufre, ou par une ou plusieurs fonctions carbonyles,
le groupement R₂ représente un atome d'hydrogène ou d'halogène, un radical choisi parmi alkyle, alcoxy, alcoxy alkyle, hydroxy alcoxy ou mono- ou poly-hydroxy alkyle,
n représente un entier variant de 1 à 4,
ou ses sels d'addition
à l'exception des composés suivants :

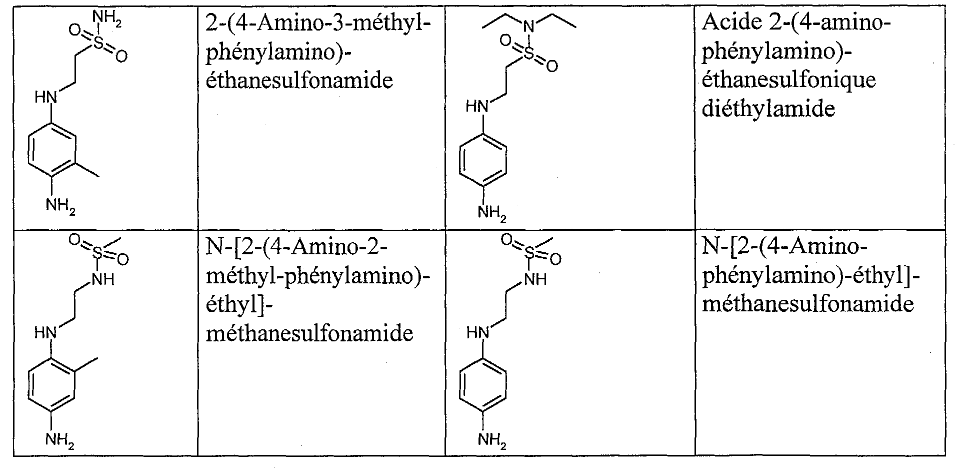

Les composés plus particulièrement préférés sont :

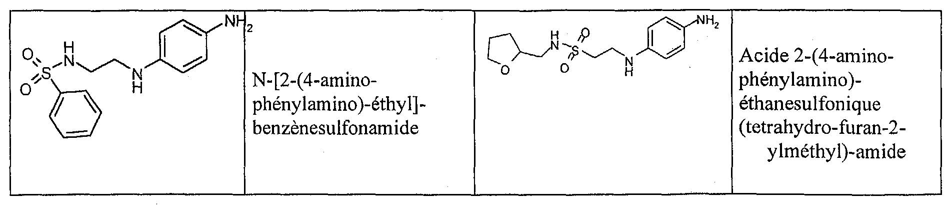

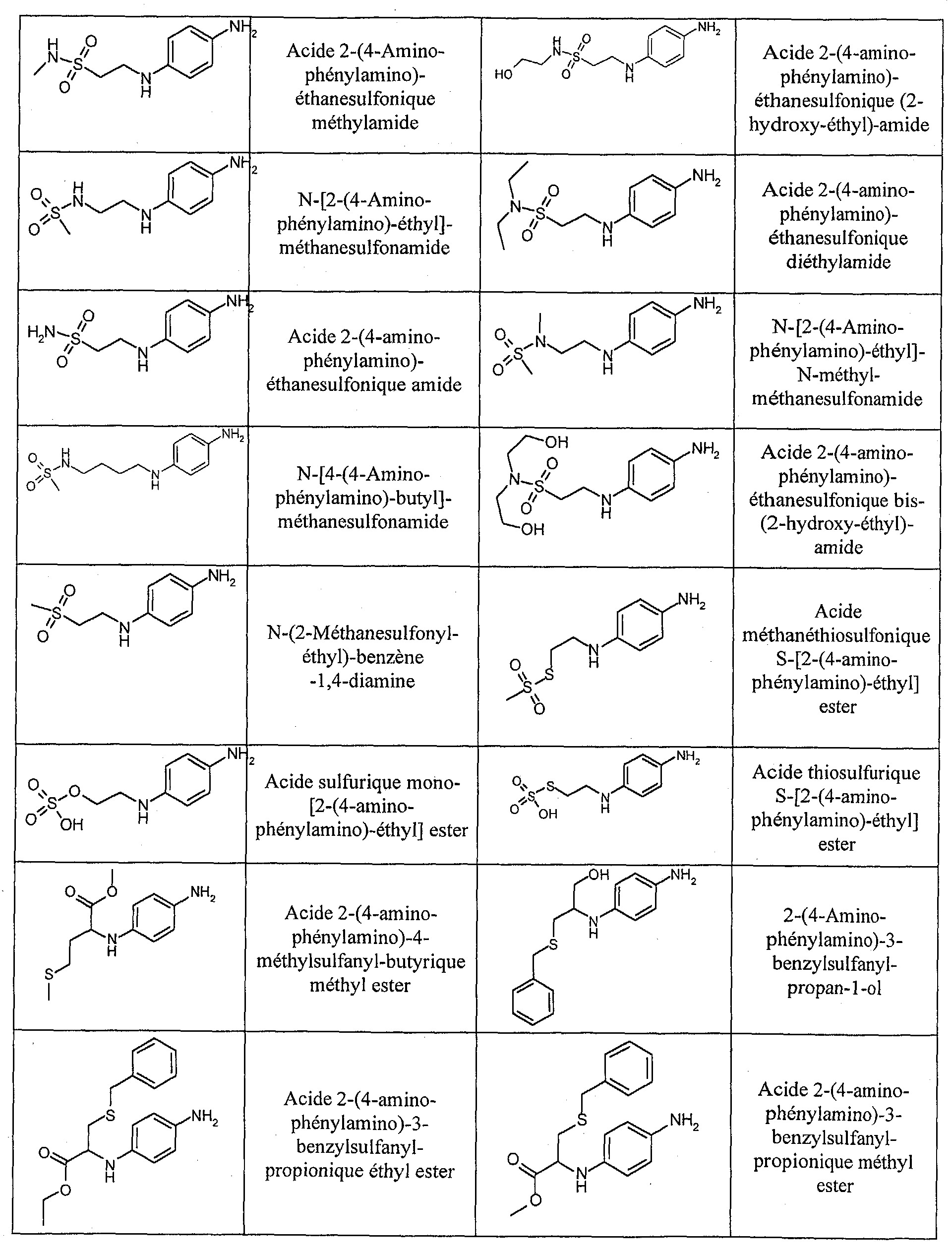

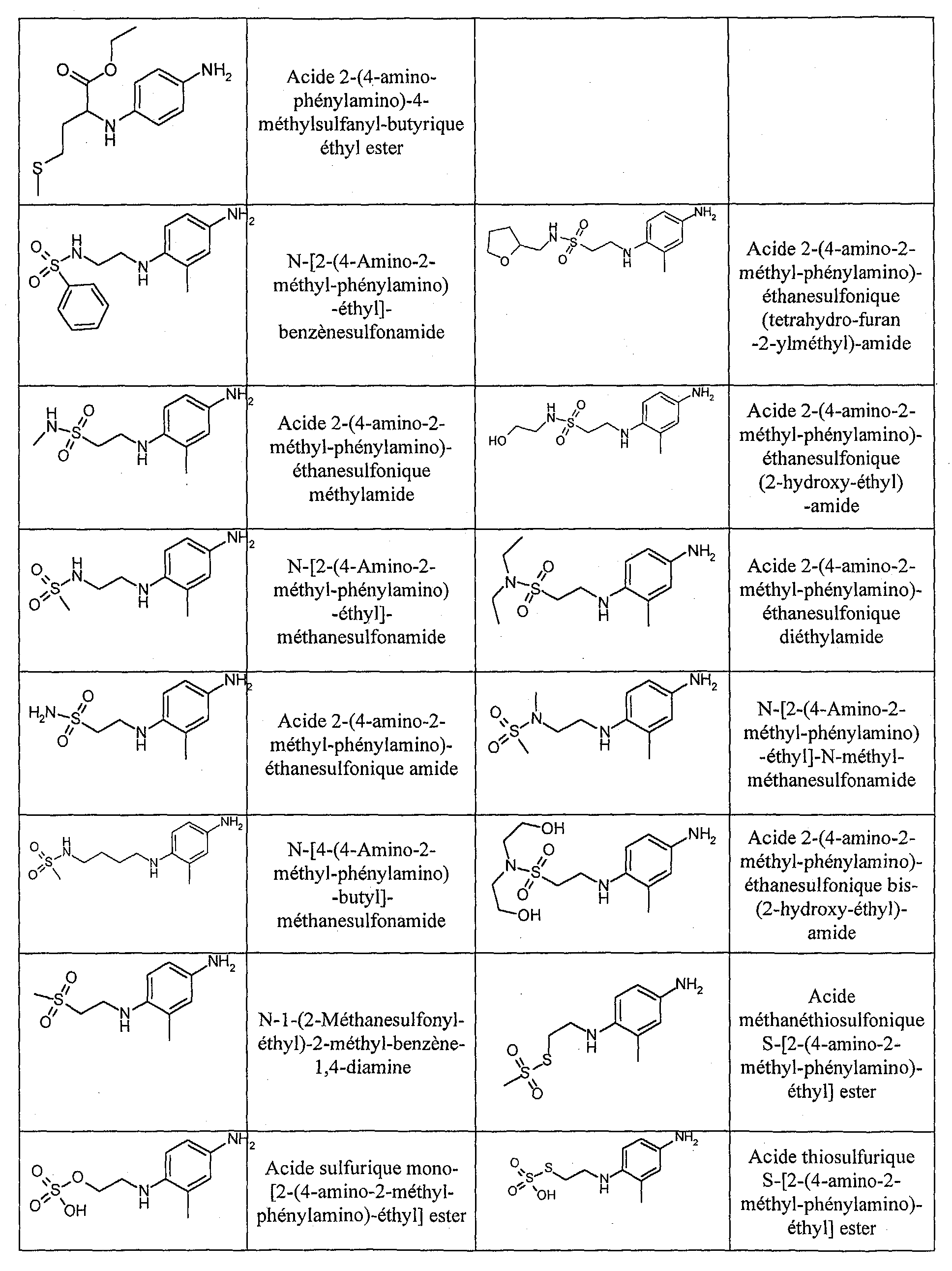

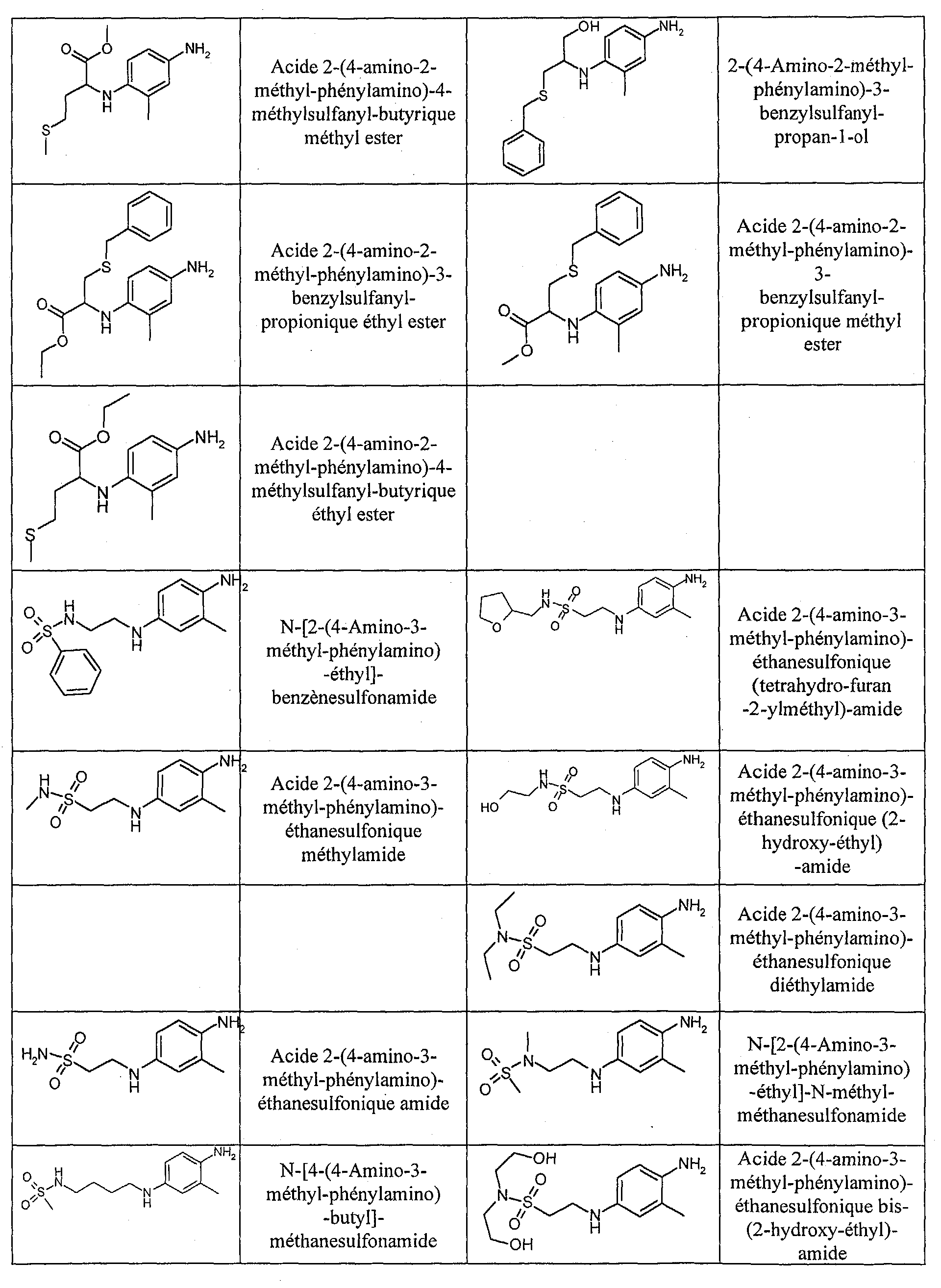

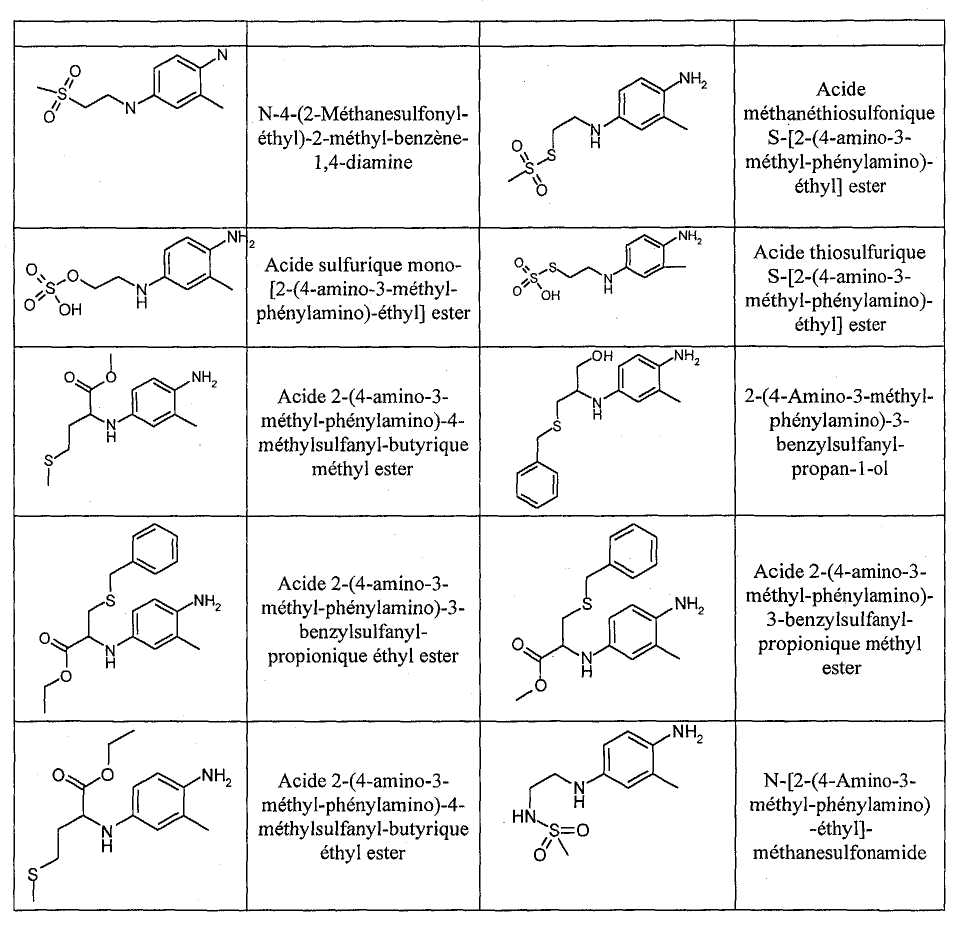

De préférence, la concentration du composé de formule générale (I) est comprise entre 0,0001 et 20 %, de préférence entre 0,005 à 6 % en poids par rapport au poids total de la composition.

Le milieu approprié pour la teinture est avantageusement constitué par de l'eau ou par un mélange d'eau et d'au moins un solvant organique tel que, par exemple, les alcools inférieurs en C₁-C₄, ramifiés ou non, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, le glycérol ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition cosmétique conforme à l'invention comprend au moins un adjuvant cosmétique choisi dans le groupe formé par les agents anti-oxydants, les agents de pénétration, les agents séquestrants, les parfums, les tampons, les agents dispersants, les tensioactifs, les agents de conditionnement, les agents filmogènes, les polymères, les céramides, les agents conservateurs, les agents nacrants ou opacifiants, les vitamines ou provitamines.

Les adjuvants ci-dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition de la présente invention contient de préférence au moins un coupleur d'oxydation. Parmi les coupleurs d'oxydation, on peut notamment citer les méta-phénylènediamines, les méta-aminophénols, les méta-diphénols, les coupleurs naphtaléniques et les coupleurs hétérocycliques ainsi que leurs sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène (ou résorcinol), le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6-hydroxy benzomorpholine, la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

Généralement la concentration du ou des coupleurs d'oxydation est comprise entre 0,0001 et 20 %, de préférence entre 0,005 à 6 % en poids par rapport au poids total de la composition.

La composition de la présente invention contient de préférence au moins un précurseur de colorant d'oxydation additionnel différent des composés de formule (I).

Les bases d'oxydation additionnelles peuvent notamment être choisies parmi les para-phénylènediamines, les bis-phénylalkylènediamines, les para-aminophénols, les ortho-aminophénols, les bases hétérocycliques et leurs sels d'addition.

Parmi les para-phénylènediamines, on peut plus particulièrement citer à titre d'exemple, la para-phénylènediamine, la para-toluènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N -(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy para-phénylènediamine, la N-(β-méthoxyéthyl) para-phénylènediamine, la 4 aminophényl pyrrolidine, le 2 thiényl para-phénylène diamine, le 2-β hydroxyéthylamino 5-amino toluène, la 3-hydroxy 1-(4' aminophényl)pyrrolidine, la 6-(4-Amino-phénylamino)-hexan-1-ol et leurs sels d'addition avec un acide.

Parmi les para-phénylènediamines citées ci-dessus, la para-phénylènediamine, la para-toluylènediamine, la 2-isopropyl para-phénylènediamine, la 2-β-hydroxyéthyl para-phénylènediamine, la 2-β-hydroxyéthyloxy para-phénylènediamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-β-acétylaminoéthyloxy para-phénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 2-méthyl phénol, le 4-amino 3-méthyl phénol, le 4-amino 3-fluoro phénol, le 4-amino 3-chloro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, le 4-amino-2,6-dichlorophénol, le 4-amino-6[((5'-amino-2'-hydroxy-3'-méthyl)phényl)méthyl]-2-méthylphénol, le bis(5'-amino-2'-hydroxy)phénylméthane et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3 -amino-pyrazolo [1 ,5-a]pyridine-5-yl)-éthanol ; le 2-(3-amino-pyrazolo[l,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3 ,4-diamino-pyrazolo [1,5 -a]pyridine ; la pyrazolo[1,5-a]pyridine-3,7-diamine ; la 7-morpholin-4-yl-pyrazolo[1,5-a]pyridin-3-ylamine ; la pyrazolo [1,5 -a]pyridine-3, 5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ;
ainsi que leurs sels d'addition avec un acide.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition.

Généralement la concentration de la ou des bases d'oxydation additionnelle est comprise entre 0,0001 et 20 %, de préférence entre 0,005 à 6% en poids par rapport au poids total de la composition.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates.

La composition tinctoriale conforme à l'invention peut en outre contenir un ou plusieurs colorants directs pouvant notamment être choisis parmi les colorants nitrés de la série benzénique, neutres, acides ou cationiques, les colorants directs azoïques neutres acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels. De préférence, la composition selon l'invention comprend au moins un colorant choisi parmi les colorants directs cationiques et les colorants directs naturels.

Parmi les colorants directs cationiques utilisables selon l'invention, on peut citer les colorants directs azoïques cationiques décrits dans les demandes de brevets WO 95/15144, WO-95/01772 et EP-714954.

Parmi ces composés on peut tout particulièrement citer les colorants suivants :
- chlorure de 1,3-diméthyl-2-[[4-(diméthylamino)phényl]azo]-1H-Imidazolium,
- chlorure de 1,3-diméthyl-2-[(4-aminophényl)azo]-1H-Imidazolium,
- méthylsulfate de 1-méthyl-4-[(méthylphénylhydrazono) méthyl]-pyridinium.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Le ou les colorants directs représentent de préférence de 0,001 à 20 % en poids environ du poids total de la composition prête à l'emploi et encore plus préférentiellement de 0,005 à 10 % en poids environ.

Une composition tinctoriale prête à l'emploi est obtenue par ajout d'un ou plusieurs agents oxydants. Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases, le peroxyde d'hydrogène étant particulièrement préféré, à la composition tinctoriale précédemment décrite.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques autres que les diacides carboxyliques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule: 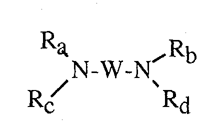 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de la présente demande concerne un procédé dans lequel on applique sur les fibres la composition selon la présente invention telle que définie précédemment, et qu'on révèle la couleur à l'aide d'un agent oxydant. La couleur peut être révélée à pH acide, neutre ou alcalin. L'agent oxydant peut être ajouté à la composition de l'invention juste au moment de l'emploi. Il peut être mis en oeuvre à partir d'une composition oxydante le contenant, appliquée simultanément ou séquentiellement sur les fibres au moment de l'application de la composition tinctoriale.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

La présente demande concerne également l'utilisation de la composition cosmétique selon l'invention comprenant, dans un milieu approprié pour la teinture, au moins un composé de formule générale (I) pour la teinture des fibres, de préférence les fibres kératiniques telles que les cheveux.

L'invention a aussi pour objet un dispositif à plusieurs compartiments ou "kit" de teinture dans lequel un premier compartiment renferme la composition tinctoriale définie ci-dessus et un deuxième compartiment renferme une composition oxydante. Ce dispositif peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de teindre les fibres kératiniques à partir d'un procédé qui comprend le mélange d'une composition tinctoriale conforme à l'invention avec un agent oxydant tel que défini précédemment, et l'application du mélange obtenu sur les fibres kératiniques pendant un temps suffisant pour développer la coloration désirée.

Les exemples qui suivent servent à illustrer l'invention sans toutefois présenter un caractère limitatif.

### EXEMPLES

### Exemple 1 : Synthèse du N-{2-[(4-aminophényl)amino]éthyl}benzène sulfonamide (3)

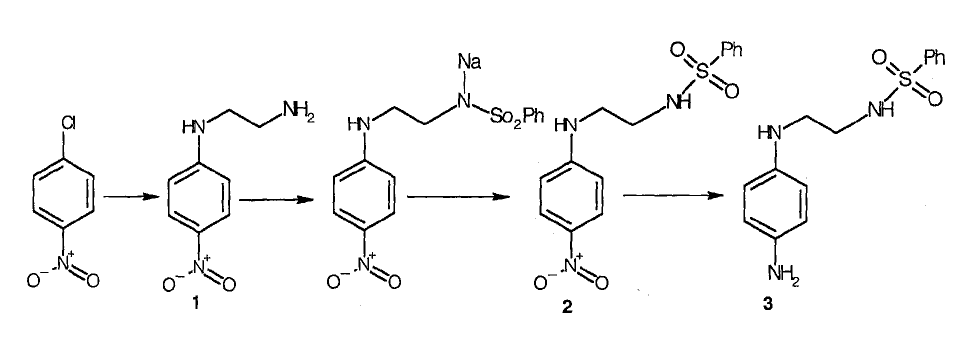

### Etape 1 : Préparation du N-(2-aminoéthyl)-N-(4-aminophényl)amine (1)

Dissoudre le para-chloronitrobenzène (181,6g, 1,152 mole) dans 180ml de pyridine en tiédissant. Couler en 1h15 et au goutte à goutte cette solution dans un ballon contenant l'éthylène diamine (359,4g, 4,608 moles) sous agitation. Porter le mélange à 90°C pendant 2 heures au bain-marie bouillant. Verser la solution sur 3L d'eau sous agitation. Laisser refroidir à la température ambiante et essorer le précipité puis re-empâter deux fois dans l'eau. Le produit brut est repris dans 1200 mL d'acide chlorhydrique 2N pendant 1 heure. Filtrer le chlorhydrate et le dissoudre dans 2L d'eau à 95°C et filtrer à nouveau l'insoluble. La solution aqueuse de chlorhydrate obtenue est versée chaude dans un bécher, vers 80°C. Additioner 120ml de soude 0,5N jusqu'à pH=12. Après refroidissement le produit cristallisé est essoré, ré-empaté à l'eau puis recristallisé dans l'éthanol absolu. On isole 155g de solides oranges (fusion = 149°C).

### Etape 2 : Préparation du N-{2-[(4-nitrophényl)amino]éthyl}benzène sulfonamide (2)

Dans un ballon de 500ml, mettre en suspension 45,3g (0,25 mole) de N-(2-aminoéthyl)-N-(4-aminophényl)amine (1) dans 200 mL de pyridine. Porter ce mélange à 45-50°C et couler en 15 minutes le chlorure de benzène sulfonyle (48,6g, 35,2 mL). Laisser la solution obtenu encore 1h30 à 50°C puis verser sur 1kg de glace et 143 mL d'acide chlorhydrique concentré en agitant. Le solide jaune formé est essoré puis ré-empâté dans l'eau pour donner 136g (humide) de produit jaune. Le produit est traité avec 650 ml de soude 1N à 85°C. Filtrer à chaud et laisser refroidir le filtrat pour essorer le produit formé. Après séchage, on obtient 81 g de produit (sel de sodium correspondant, fusion = 170°C).
Pour libérer le sel de sodium, on dissout le produit (60g) dans 300 ml de soude 1N à 85°C, après filtration à chaud on neutralise le filtrat avec de l'acide chlorhydrique 5N jusqu'à pH=3. Le produit cristallisé est essoré, lavé à l'eau puis séché sur P₂O₅ pour donner 50 g de produit attendu avec un point de fusion de 171 °C.

### Etape 3 : Préparation du N-{2-[(4-aminophényl)amino]éthyl} benzènesulfonamide (3)

Le mélange de zinc en poudre (2g) et de chlorure d'ammonium (0,2g) dans 22 ml d'un mélange eau/éthanol (1/1) est porté au reflux au bain-marie bouillant. Le dérivé N-{2-[(4-nitrophényl)amino]éthyl} benzènesulfonamide (2) (3g, 9 mmoles) est ajouté petit à petit et le chauffage est maintenu jusqu'à décoloration du milieu réactionnel. On filtre à chaud et rince le zinc avec une solution éthanol/eau (50/50). Le filtrat cristallise rapidement. Après refroidissement à 0°C, le solide est filtré puis lavé à l'eau. Après séchage, on obtient 2,27g de produit brut (Fusion = 144°C). 0,77g de ce brut est recristallisé dans 20 mL d'eau/éthanol (1/1), les cristaux obtenus sont séchés sur P₂O₅ et sont microanalysés.

| | **THEORIE** | **TROUVE** |
|---|---|---|
| C | 57.72 | 57.90 |
| H | 5.88 | 5.90 |
| N | 14.43 | 14.22 |
| S | 10.98 | 11.02 |

Les spectres RMN du proton et de masse sont conformes à la structure attendue du produit.

### Exemple 2 : Synthèse du 2-[(4-aminophényl)amino]-N(tétrahydrofuran-2-ylméthyl)éthanesulfonamide, dichlorhydrate (11)

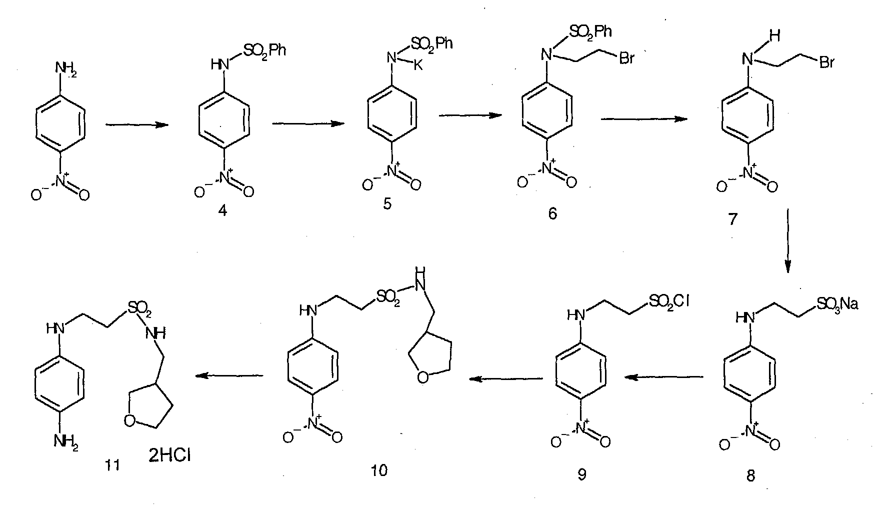

### Etape 1 : Préparation du N-(4-Nitro-phényl)-benzènesulfonamide (4)

La solution de p-nitroaniline (1 kg, 7,245 moles) dans la pyridine (3,6L) est portée à 45°C. On coule le sulfochlorure de benzène (1,41 kg, 8 moles) sur 1h10 en maintenant la température entre 45-50°C. Après 1 heure à 50°C, verser le milieu réactionnel sur 8kg de glace et 4,35L d'acide chlorhydrique. Le précipité jaune formé est essoré puis dissous dans 20L de soude 0,5 N à 55°C. L'insoluble est éliminé par filtration, puis neutraliser le filtrat encore chaud avec de l'acide chlorhydrique. Le précipité jaune formé est essoré et ré-empâté dans l'eau puis séché à l'air. 2 kg de produits sont obtenus (fusion= 137°C).

### Etape 2: Préparation du sel de potassium du N-(4-Nitro-phényl)-benzènesulfonamide (5)

1,817kg (6,5 moles) de N-(4-Nitro-phényl)-benzènesulfonamide sont mis en solution dans 4,5L d'éthanol puis chauffés à reflux. 470g d'hydroxyde de potassium (pur 85%, 7,15 moles) sont solubilisés dans 250 ml d'eau et cette solution est ajoutée en 30 min dans le milieu réactionnel vers 75°C. Après refroidissement, le composé (5) cristallise. Ce produit est essoré, lavé à l'éthanol puis à l'éther de pétrole. Après séchage, on obtient 1,85 kg de produit (5).

### Etape 3 : Préparation du N-(2-Bromo-éthyl)-N-(4-nitro-phényl)-benzène sulfonamide (6)

Le 1,2-dibromoéthane (2,26 kg, 12 moles) mélangé à 4L de diméthylformamide est porté à 80°C . Ajouter par portion 1,264 kg (4 moles) de sel de potassium de N-(4-Nitro-phényl)-benzènesulfonamide et laisser agiter 4h vers 90-95°C. Filtrer à chaud, laver le sel KBr formé avec la diméthylformamide. Le filtrat est versé sur 20kg de glace + 20 kg d'eau. Le précipité jaune formé est essoré, ré-empâté 3 fois dans la soude 1N chaude puis ré-empâté dans l'eau jusqu'à pH neutre. Le produit brut (1,2kg humide) obtenu est recristallisé dans 3L d'acide acétique. On filtre bouillant et le filtrat est refroidi à 20°C. Le précipité blanc formé est essoré, lavé à l'acide acétique puis à l'éther de pétrole puis séché sous vide. Le produit est isolé (773g) et fond à 168°C.

### Etape 4 : Préparation du (2-Bromo-éthyl)-(4-nitro-phényl)-amine (7)

Solubiliser 770 g (2 moles) de produits obtenus ci-dessus (6) dans 3,85L d'acide sulfurique. Laisser agiter 1 heure à température ambiante et verser sur 20 kg de glace +10 kg d'eau. Filtrer le précipité jaune et ré-empâter dans l'eau jusqu'à pH neutre et laisser sécher. 482 g de produits sont obtenus (Fusion = 95°C).

### Etape 5 : Préparation du 2-[(4-nitro-phényl)-amino]-éthanesulfonate de sodium (8)

661 g (2,7 moles) de (2-Bromo-éthyl)-(4-nitro-phényl)-amine sont partiellement solubilisés dans 3,7L d'éthanol 96%. Vers 75-80°C, on coule une solution aqueuse de sulfite de sodium (820 g, 6,5 moles de Na₂SO₃ dans 2,4L d'eau). Laisser agiter au reflux pendant 11 heures. Filtrer bouillant, laisser refroidir le filtrat puis essorer le précipité jaune formé et le laver à l'acétone. Après séchage, 623g de produits sont isolés.

### Etape 6 : Préparation du chlorure de 2-[(4-nitro-phényl)amino]-éthanesulfonyle (9)

Le mélange de 2-[(4-nitro-phényl)-amino]-éthanesulfonate de sodium (5,36g, 0,02 mole), le pentachlorure de phosphore (4,58g, 0,022 mole) et 50 ml de xylène anhydre est chauffé vers 40-50°C pendant 4 heures. Après 1 nuit à température ambiante, on chauffe 3 heures au bain-marie bouillant. Filtrer à chaud, laver l'insoluble 3 fois au xylène chaud. Laisser refroidir le filtrat, filtrer le solide jaune formé, le ré-empâter dans l'eau. 2,4g de produits attendus ont été isolés.
Les résultats d'analyses élémentaires sont les suivants :

| | **THEORIE** | **TROUVE** |
|---|---|---|
| C | 36.29 | 36.48 |
| H | 3.40 | 3.59 |
| N | 10.58 | 10.48 |
| Cl | 13.42 | 13.59 |
| S | 12.10 | 12.06 |

### Etape 7 : Préparation du 2-[(4-nitro-phényl)amino]-N(tétrahydrofuran-2-ylméthyl)éthanesulfonamide (10)

Mélanger 20 ml (0,2 mole) de tétrahydrofurfurylamine dans 75 mL d'acétate d'éthyle. A 0°C, introduire le chlorure de 2-[(4-nitro-phényl)amino]-éthanesulfonyle (13,2g, 0,05 mole) puis laisser revenir à température ambiante pendant la nuit. Filtrer le chlorhydrate d'amine formé et extraire la phase d'acétate d'éthyle à l'acide chlorhydrique 2,5N puis à l'eau. Chasser la phase organique et évaporer à sec sous vide pour obtenir 13,5g de produit sous forme d'huile.

### Etape 8 : Préparation du 2-[(4-aminophényl)amino]-N(tétrahydrofuran-2-ylméthyl)éthanesulfonamide, dichlorhydrate (11)

Le mélange de zinc en poudre (3,8g), le chlorure d'ammonium (0,22g), l'eau (0,75 mL) et l'éthanol 96% (10 mL) est porté au reflux au bain-marie bouillant. Le dérivé 2-[(4-nitro-phényl)amino]-N(tétrahydrofuran-2-ylméthyl)éthanesulfonamide (2,52g, 7,6 mmoles) est ajouté petit à petit et le chauffage continue jusqu'à décoloration du milieu réactionnel. On filtre bouillant et le filtrat est récupéré sur 12 mL d'éthanol et 1,5 mL d'acide chlorhydrique concentré. Ajouter de l'acétone pour précipiter le chlorhydrate formé (1,8g). Filtrer et recristalliser dans 15 mL d'éthanol pour donner 0,8g de produit dichlorhydraté avec les analyses élémentaires suivantes :

| | **THEORIE, 2HCl** | **TROUVE** |
|---|---|---|
| C | 41.94 | 42.07 |
| H | 6.23 | 6.39 |
| N | 11.29 | 11.11 |
| Cl | 19.05 | 19.32 |
| S | 8.60 | 8.61 |

Les spectres RMN du proton et de masse sont conformes à la structure attendue du produit.

### Exemple 3 : Synthèse du 2-(4-Amino-phénylamino)-éthane sulfonamide (13)

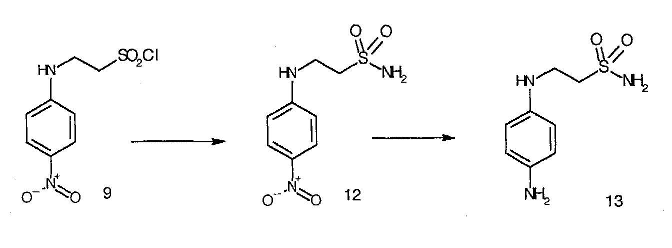

### Etape 1 : Préparation du 2-(4-Nitro-phénylamino)-éthanesulfonamide (12)

Mélanger le chlorure de 2-[(4-nitro-phényl)amino]-éthanesulfonyle (9) (0,5g) avec 4 mL d'ammoniaque (22%) à la température ambiante. Laisser 1 heure puis essorer le précipité formé et ré-empâter dans l'eau. Le brut est recristallisé dans l'éthanol et donne 0,23g (Fusion = 170°C) de produit attendu dont l'analyse élémentaire est la suivante :

| | **THEORIE** | **TROUVE** |
|---|---|---|
| C | 39.17 | 38.61 |
| H | 4.52 | 4.86 |
| N | 17.13 | 16.12 |
| S | 13.07 | 12.91 |

### Etape 2 : Préparation du 2-(4-Amino-phénylamino)-éthanesulfonamide (13)

Mettre en suspension 1g de 2-(4-Nitro-phénylamino)-éthanesulfonamide (12) dans 10 mL d'éthanol. A température ambiante, verser sur la solution réductrice d'hydrosulfite de sodium (8g) - soude 1N (30mL). Après décoloration, un précipité se forme. 0,7 g de produit brut est isolé par essorage et ré-empâtage dans l'eau.

Les analyses élémentaires sont les suivantes :

| | **THEORIE** | **TROUVE** |
|---|---|---|
| C | 44.63 | 43.74 |
| H | 6.09 | 6.18 |
| N | 19.52 | 18.59 |
| S | 14.89 | 14.27 |

Les spectres RMN du proton et de masse sont conformes à la structure attendue du produit.

### Exemple 4 : Synthèse du 2-(4-Amino-phénylamino)-éthanesulfonyl méthylamide (15)

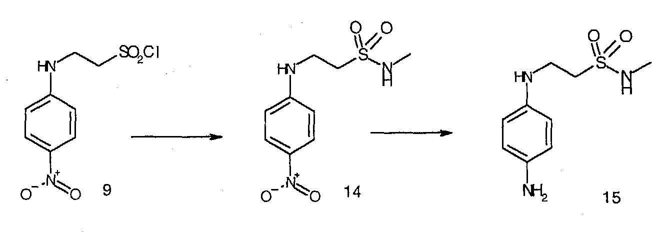

### Etape 1 : Préparation du 2-(4-Nitro-phénylamino)-éthanesulfonyl méthylamide (14)

Refroidir 5 ml de monométhylamine (solution aqueuse de 40%) dans un bain de glace et ajouter par fraction le 2-[(4-nitro-phényl)amino]-éthanesulfonyle (9) (2,6g, 0,01 mole). Un précipité jaune se forme et le mélange est abandonné une nuit à la température ambiante. Essorer, ré-empâter dans l'eau et sécher. Le produit brut (2g) est recristallisé dans l'éthanol et 1,57g sont obtenus après séchage (Fusion = 130°C) avec les analyses élémentaires suivantes :

| | **THEORIE** | **TROUVE** |
|---|---|---|
| C | 41.70 | 71.80 |
| H | 5.06 | 5.40 |
| N | 16.21 | 16.32 |
| S | 12.34 | 12.34 |

### Etape 2 : Préparation du 2-(4-Amino-phénylamino)-éthanesulfonyl méthylamide (15)

Le mélange de zinc en poudre (25g), de chlorure d'ammonium (1,5g), d'eau (5 mL) et d'éthanol 96% (70 mL) est porté au reflux au bain-marie bouillant. Le dérivé 2-(4-Nitro-phénylamino)-éthanesulfonyl méthylamide (14) (13g, 0,05 mole) est ajouté petit à petit et le chauffage continue jusqu'à décoloration du milieu réactionnel. On filtre bouillant et le filtrat est récupéré sur 10 mL d'acide chlorhydrique concentré. Laver le zinc avec de l'éthanol chaud, abandonner le filtrat au refrigérateur 1 jour. 6,11 g de produits ont été isolés avec les analyses élémentaires suivantes :

| | **THEORIE, 2HCl** | **TROUVE** |
|---|---|---|
| C | 35.77 | 35.73 |
| H | 5.67 | 5.85 |
| N | 13.90 | 13.94 |
| Cl | 23.47 | 23.33 |

Les spectres RMN du proton et de masse sont conformes à la structure attendue du produit.

### Exemple 5 : Synthèse du 2-(4-Amino-phénylamino)-éthanesulfonyl 2-hydroxyéthylamide (17)

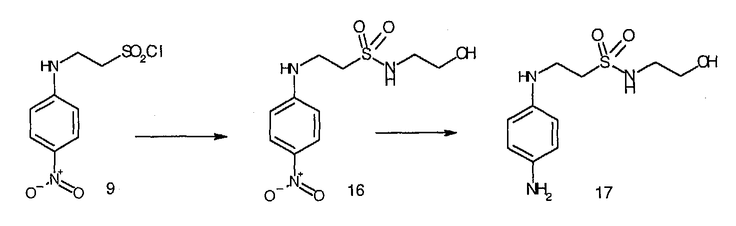

### Etape 1 : Préparation du 2-(4-Nitro-phénylamino)-éthanesulfonyl (2-hydroxy-éthyl)-amide (16)

Refroidir 30g d'éthanolamine dans un bain de glace, ajouter par fraction 13g (0,05 mole) de 2-[(4-nitro-phényl)amino]-éthanesulfonyle (9). Laisser à la température ambiante pendant 40 heures et verser dans 200 ml d'acide chlorhydrique 2,5N froid. Extraire à l'acétate d'éthyle, laver la phase organique à l'acide chlorhydrique puis à l'eau. Sécher et concentrer la phase d'acétate d'éthyle jusqu'à 20 mL puis laisser cristalliser le produit. Après essorage et séchage, on isole 7,73g (Fusion = 114°C) de produit attendu.

### Etape 2 : Préparation du 2-(4-amino-phénylamino)-éthanesulfonyl (2-hydroxy-éthyl)-amide (17)

Le mélange de zinc en poudre (5g), le chlorure d'ammonium (0,3g), l'eau (1 mL) et l'éthanol 96% (14 mL) est porté au reflux au bain-marie bouillant. Le dérivé 2-(4-Nitro-phénylamino)-éthanesulfonyl (2-hydroxy-éthyl) amide (16) (2,89g, 0,01 mole) est ajouté petit à petit et le chauffage continue jusqu'à décoloration du milieu réactionnel (1/2 h). On filtre bouillant et le filtrat est récupéré sur 2 ml d'acide chlorhydrique concentré. Laver le zinc avec 5 d'éthanol, refroidir le filtrat dans la glace et essorer le précipité formé m = 1,88g. Après recristallisation dans 30 mL d'éthanol, on isole 1,5g de composé attendu avec les analyses élémentaires suivantes :

| | **THEORIE, 1HCl** | **TROUVE** |
|---|---|---|
| C | 40.61 | 40.47 |
| H | 6.13 | 6.13 |
| N | 14.21 | 14.08 |
| Cl | 11.99 | 12.20 |
| S | 10.82 | 10.60 |

Les spectres RMN du proton et de masse sont conformes à la structure attendue du produit.

### Exemple 6 : Synthèse du 2-(4-Amino-phénylamino)-éthanesulfonamide, dichlorhydrate (20)

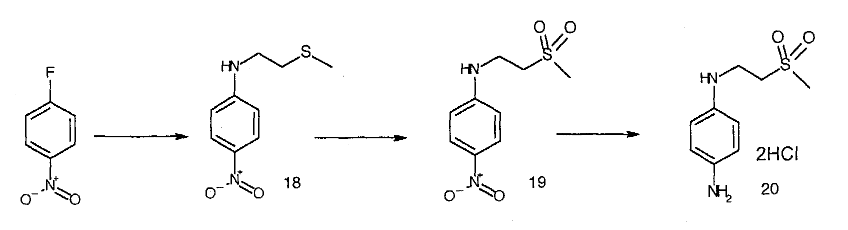

### Etape 1 : Préparation du (2-Méthylsulfanyl-éthyl)-(4-nitro-phényl)-amine (18)

Mélanger le 2-aminoéthanéthiol dichlorhydrate (45,4g, 0,4 mole), l'eau (100 mL) et 56,8g (0,4 mole) d'iodure de méthyle. Dissoudre 16,5g de pastille de soude (0,4 mole) dans 100mL d'eau et verser cette solution au goutte à goutte dans le milieu réactionnel en maintenant la température entre 27-30°C puis laisser agiter 1 heure à 40°C. Ajouter 21,2g (10,2 mole) de parafluoronitrobenzène puis 42 mL de triéthylamine et porter au bain-marie bouillant pendant 3 heures. Laisser 1 nuit à la température ambiante puis acidifier à l'acide chlorhydrique et extraire avec l'acétate d'éthyle. Après évaporation à sec, les 30 g d'huile obtenus sont dissous dans 100ml d'HCl 36%. Extraire avec l'éther éthylique pour éliminer le produit de départ et, à froid, neutraliser la phase aqueuse avec l'ammoniaque. Extraire à nouveau avec de l'éther éthylique, sécher et évaporer à sec pour obtenir 11g d'huile.
La purification par chromatographie sur silice a permis d'isoler 20g de produit attendu pur avec les analyses élémentaires suivantes :

| | **THEORIE** | **TROUVE** |
|---|---|---|
| C | 50.94 | 51.10 |
| H | 5.70 | 5.74 |
| N | 13.20 | 13.25 |
| O | 15.08 | 15.20 |
| S | 15.08 | 15.15 |

### Etape 2 : Préparation du (2-Méthanesulfonyl-éthyl)-(4-nitro-phényl)-amine (19)

8,4g (0,04 mole) de (2-Méthylsulfanyl-éthyl)-(4-nitro-phényl)-amine (18) sont dissous dans 40 mL d'acide acétique à la température ambiante. Ajouter au goutte à goutte 18,7 mL (0,16 mole) d'eau oxygénée (solution à 30%) en maintenant la température à 40-45 °C avec un bain de glace. Chauffer progressivement au bain-marie bouillant jusqu'à atteindre 95°C. Laisser 45 minutes puis verser sur 80 mL d'eau glacée. Essorer les cristaux bruns, sécher puis recristalliser dans 120 mL d'éthanol 96%. 4,6 g de produits ont été isolés puis repris dans 50 ml d'acide chlorhydrique concentré et 5 ml d'eau. Filtrer l'insoluble et neutraliser en refroidissant le filtrat avec l'ammoniaque. Essorer les cristaux bruns orangés et sécher sur P₂O₅ (m= 3,2g, Fusion = 149°C). Recristalliser dans 120 ml d'acétate d'éthyle pour donner 2,3 g de cristaux jaunes (Fusion = 150°C) avec les analyses élémentaires suivantes :

| | **THEORIE** | **TROUVE** |
|---|---|---|
| C | 44.26 | 44.29 |
| H | 4.95 | 4.97 |
| N | 11.47 | 11.45 |
| O | 26.20 | 26.00 |
| S | 13.11 | 13.05 |

### Etape 3 : Préparation du N-(2-Méthanesulfonyl-éthyl)-benzène-1,4-diamine, dichlorhydrate (20)

Solubiliser 1,8 g (7,37 mmole) de (2-Méthanesulfonyl-éthyl)-(4-nitro-phényl)-amine (19) dans 70 mL d'éthanol absolu. La solution est hydrogénée pendant 1h30 sous 60 bars d'hydrogène à 50 °C en présence de 1,5 g de palladium sur charbon à 10%. Filtrer le catalyseur et récupérer le filtrat sur 5 mL d'éthanol chlorhydrique 6,4N. Refroidir, essorer, sécher sur P₂O₅/KOH pour donner 1,4g de cristaux blancs.

Les spectres RMN du proton et de masse sont conformes à la structure attendue du produit.

### Exemple 7 : Synthèse du N-[2-(4-Amino-phénylamino)-éthyl]-N-méthyl-méthanesulfonamide, sulfate (24)

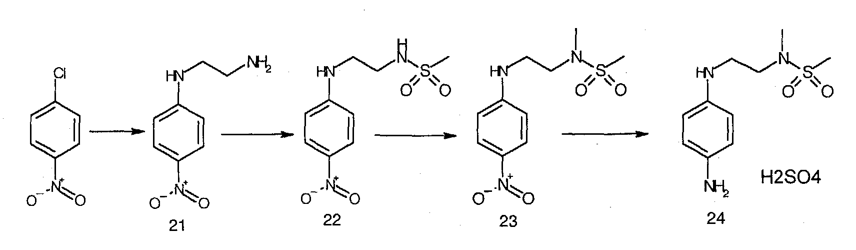

### Etape 1 : Préparation du N-1-(4-Nitro-phényl)-éthane-1,2-diamine (21)

On chauffe au bain-marie bouillant 1382 g (23 moles) d'éthylènediamine puis on y ajoute en 15 min une solution de parachloronitrobenzène (906g, 5,75 moles dans 900g de pyridine) tout en maintenant le chauffage pendant 2 heures. Lorsque la réaction est totale, le milieu est versé sur 15 kg d'eau glacée. Le précipité jaune formé est essoré et ré-empâté 3 fois dans l'eau. Le produit brut obtenu est solubilisé dans 6L d'acide chlorhydrique 2N, laisser agiter pendant 1 heure puis filtrer le chlorhydrate. Dissoudre le produit obtenu dans 12L d'eau bouillante. Filtrer à chaud les traces de résidu et refroidir à 80°C pour alcaliniser avec de l'ammoniaque jusqu'à pH=12. Le précipité jaune formé est filtré à froid, lavé puis recristallisé dans 3L d'éthanol à 96%. Après séchage sous vide, on obtient 716g de produit attendu avec un point de fusion de 150°C.

### Etape 2 : Préparation du N-[2-(4-Nitro-phénylamino)-éthyl]-méthanesulfonamide (22)

90,5g (0,5 mole) de N-1-(4-Nitro-phényl)-éthane-1,2-diamine (21) sont mis en suspension dans 400ml de pyridine. Chauffer à 45°C et couler 63g (0,55 mole) de chlorure de mésyle en maintenant la température entre 45-50°C. La solution obtenue est laissée agitée 1 heure à 50°C. Verser sur 2kg de glace et 300 ml d'acide chlorhydrique concentré en agitant. Essorer le précipité jaune et ré-empâter 3 fois dans l'eau et sécher. On obtient 129 g de poudre jaune (Fusion = 154°C) avec un rendement quantitatif.

### Etape 3 : Préparation du N-Méthyl-N-[2-(4-Nitro-phénylamino)-éthyl]-méthanesulfonamide (23)

25,9 g (0,1 mole) de N-[2-(4-Nitro-phénylamino)-éthyl]-méthanesulfonamide (22) sont dissous dans 200 ml de soude 1N à 50°C. Filtrer l'insoluble, laisser refroidir à 30°C et ajouter au goutte à goutte 11,4 ml (0,12 mole) de sulfate de méthyle. Laisser agiter 1 heure à température ambiante puis chauffer à 40°C et filtrer le précipité jaune. Ré-empâter dans la soude 0,5N et dans l'eau. Le produit brut humide est recristallisé dans 50 ml d'éthanol 96% avec du noir de charbon. 13,3 g de produit ont été isolés (Fusion = 109°C).

| | **THEORIE** | **TROUVE** |
|---|---|---|
| C | 43.95 | 44.12 |
| H | 5.53 | 5.76 |
| N | 15.38 | 15.13 |
| O | 11.77 | 11.98 |

Les spectres RMN du proton et de masse sont conformes à la structure attendue du produit.

### Etape 4 : Préparation du N-[2-(4-Amino-phénylamino)-éthyl]-N-méthyl-méthanesulfonamide, sulfate (24)

Le mélange de zinc en poudre (25g), de chlorure d'ammonium (1,1g), d'eau (11,2 ml) et d'éthanol 96% (67,5 ml) est porté au reflux au bain-marie bouillant. Le N-Méthyl-N-[2-(4-Nitro-phénylamino)-éthyl]-méthanesulfonamide (23) (12,3g, 0,045 mole) est ajouté petit à petit et le chauffage est maintenu jusqu'à décoloration du milieu réactionnel. On filtre bouillant et le filtrat est récupéré sur 3,8 ml d'acide sulfurique 96%. Laver le zinc avec de l'éthanol, refroidir le filtrat dans la glace et essorer le précipité formé. Après recristallisation dans 30 ml d'éthanol et 30 ml d'eau, on isole 14,4g de produit. Une deuxième recristallisation dans 45 ml d'éthanol 50% conduit à 11.5g de produit attendu avec les analyses élémentaires suivantes :

| | **THEORIE, H**_{**2**}**SO**_{**4**} | **TROUVE** |
|---|---|---|
| | 35.18 | 35.40 |
| | 5.61 | 5.88 |
| | 12.31 | 12.37 |
| | 18.78 | 18.71 |

Les spectres RMN du proton et de masse sont conformes à la structure attendue du produit.

### Exemple 8 : Synthèse du N-[4-(4-Amino-phénylamino)-butyl]-méthanesulfonamide, sulfate (27)

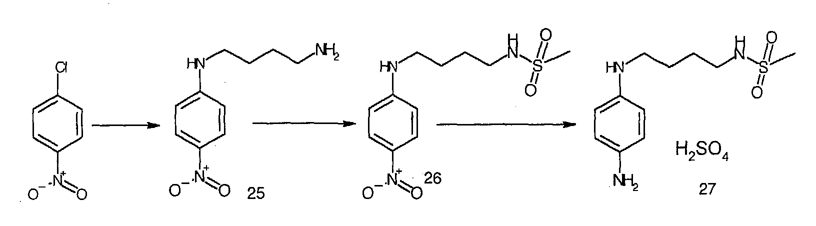

### Etape 1 : Préparation du N-1-(4-Nitro-phényl)-butane-1,4-diamine (25)

A 176,3g (2 moles) de 1,4-diaminobutane chauffés au bain-marie bouillant, on ajoute en 15 minutes une solution de 78,75g (0,5mole) de 4-chloronitrobenzène. Après 4 heures d'agitation on verse le mélange réactionnel sur 1kg de glace-eau. Le précipité est essoré, lavé à l'eau puis séché. Poids obtenu après recristallisation dans 200ml d'acétonitrile : 86,7g. F=103°C

### Etape 2: Préparation du N-[4-(4-Nitro-phénylamino)-butyl]-méthanesulfonamide (26)

Le N-1-(4-Nitro-phényl)-butane-1,4-diamine (25) obtenu dans l'étape précédente (86,5g, 0,409 mole) est solubilisé dans 340ml de pyridine à 40°C. 35,1ml (0,45mole) de chlorure de mésyle sont alors ajoutés goutte à goutte de façon à maintenir la température vers 50°C. Après 1 heure d'agitation la solution est versée sur 820g de glace et 285ml d'acide chlorhydrique concentré. Le précipité jaune est essoré, lavé à l'acide chlorhydrique puis à l'eau et est ensuite séché.
Poids obtenu après recristallisation dans l'acétonitrile : 90,6g. F=153°C

### Etape 3 : Préparation du N-[4-(4-Amino-phénylamino)-butyl]-méthanesulfonamide, sulfate (27)

25,6g (0,0891 mole) de N-[4-(4-Nitro-phénylamino)-butyl]-méthanesulfonamide (26) sont hydrogénés dans un mélange de 49g de zinc en poudre, 1,6g de chlorure d'ammonium, 24ml d'eau, 135ml d'éthanol porté au reflux. Après essorage des boues, la paraphénylène diamine est isolée sous forme de sulfate par addition de 8,1 ml d'acide sulfurique à 96%. Après filtration et séchage on obtient 31,3g de poudre grise.

Les spectres RMN du proton et de masse sont conformes à la structure attendue du produit.

### Exemple 9 : synthèse du 2-(4-Amino-phenylamino)-4-methylsulfanyl-butyric acid methyl ester (29)

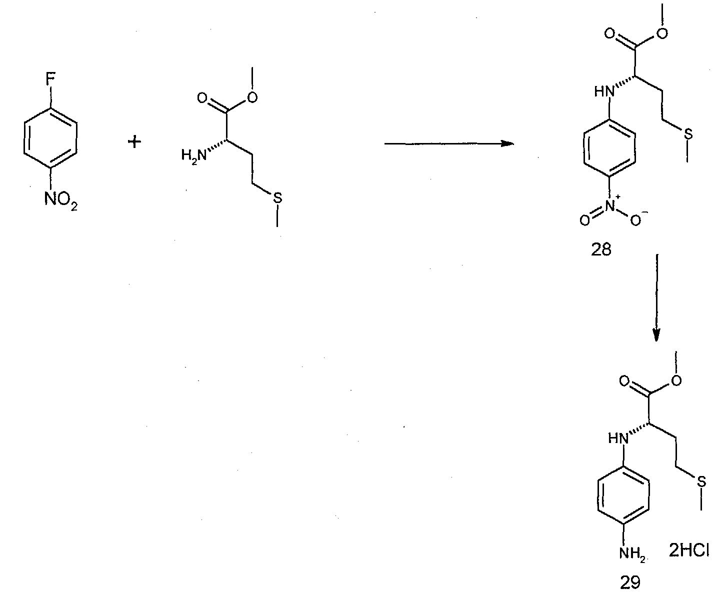

### Etape 1 : synthèse du 4-Methylsulfanyl-2-(4-nitro-phenylamino)-butyric acid methyl ester (28) :

A une solution de 5 ml de N-méthyl-pyrrolidinone, on ajoute 1 g de 4-fluoro-3-méthyl- nitrobenzène, 0,694 g de L-methioninemethylester et 1,09 ml de triéthylamine. Le milieu réactionnel est chauffé à 90°C pendant 7 heures, puis, après refroidissement à la température ambiante, est versé dans un mélange eau + glace. Le milieu réactionnel est ensuite extrait à l'acétate d'éthyle puis la phase organique est séchée et concentrée à sec. Après purification par chromatographie sur colonne de silice, on obtient le 2-[2-(4-nitro-2-méthyl-phénylamino)-éthylamino]-éthanol (28).

### Etape 2 : Synthèse du 2-(4-Amino-phenylamino)-4-methylsulfanyl-butyric acid methyl ester, dichlorhydrate (29)

Le 2-[2-(4-nitro-2-méthyl-phénylamino)-éthylamino]-éthanol (28) précédemment obtenu est réduit avec un mélange zinc / chlorure d'ammonium / eau / éthanol bouillant. L' amine correspondant est isolée sous forme de dichlorhydrate.

Les spectres RMN du proton et de masse sont conformes à la structure attendue du produit.

### EXEMPLES DE TEINTURE

### Exemples 1 à 11 : Composition tinctoriale à partir du N-{2-[(4-aminophényl)amino]éthyl}benzènesulfonamide (3)

### - Exemples 1 à 6 : Teinture en milieu acide

Les compositions tinctoriales suivantes sont préparées :

| **Exemple** | **1** | **2** | **3** | **4** | **5** | **6** |
|---|---|---|---|---|---|---|
| N-{2-[(4-aminophenyl)amino]ethyl}b enzenesulfonamide (3) | 10-3 mole | 10-3 mole | 10-3 mole | 10-3 mole | 10-3 mole | 10-3 mole |
| Benzene-1,3-diol | 10-3 mole | | | | | |
| 5-Amino-2-methyl-phenol | | 10-3 10-3 mole | | | | |
| 1H-Indol-6-ol | | | 10-3 mole | | | |
| 2-Amino-pyridin-3-ol | | | | 10-3 mole | | |
| 3,6-Dimethyl-1H-pyrazolo[5,1-c][1,2 ,4]triazole | | | | | 10-3 mole | |
| 2-(2,4-Diamino-phenoxy)-ethanol, chlorhydrate | | | | | | 10-3 mole |
| Support de teinture (1) | (*) | (*) | (*) | (*) | (*) | (*) |
| Eau déminéralisée q.s.p. | 100g | 100g | 100g | 100g | 100g | 100g |

| | | | | | | |
|---|---|---|---|---|---|---|
| (*) : support de teinture (1) pH 7 Alcool éthylique à 96° 20,8 g Métabisulfite de sodium en solution aqueuse à 35% 0,23 g M.A Sel pentasodique de l'acide diéthylène-triamine-pentaacétique en solution aqueuse à 40% 0,48 g M.A Alkyl en C8-C10 polyglucoside en solution aqueuse à 60% 3,6 g M.A Alcool benzylique 2,0 g Polyéthylène glycol à 8 motifs d'oxyde d'éthylène 3,0 g Na₂HPO₄ 0,28 g KH₂PO₄ 0,46 g | | | | | | |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| **Exemple** | **1** | **2** | **3** | **4** | **5** | **6** |
|---|---|---|---|---|---|---|
| **Nuance observée** | brun jaune | violet-rouge | brun rouge intense | brun rouge intense | rouge | bleu intense |

### - Exemples 7 à 11 : teinture en milieu basique

Les compositions tinctoriales suivantes sont préparées :

| **Exemple** | **7** | **8** | **9** | **10** | **11** |
|---|---|---|---|---|---|
| N-{2-[(4-aminophenyl)amino]ethyl }benzenesulfonamide (3) | 10-3 mole | 10-3 mole | 10-3 mole | 10-3 mole | 10-3 mole |
| 5-Amino-2-methyl-phenol | 10-3 mole | | | | |
| 1H-Indol-6-ol | | 10-3 mole | | | |
| 2-Amino-pyridin-3-ol | | | 10-3 mole | | |
| 3,6-Dimethyl-1H-pyrazolo[5,1-c][1,2 ,4]triazole | | | | 10-3 mole | |
| 2-(2,4-Diamino-phenoxy)-ethanol,chlorhydrate | | | | | 10-3 mole 10-3 mole |
| Support de teinture (2) | (*) | (*) | (*) | (*) | (*) |
| Eau déminéralisée q.s.p. | 100g | 100g | 100g | 100g | 100g |

| | | | | | |
|---|---|---|---|---|---|
| (*) : support de teinture (2) pH 9.5 Alcool éthylique à 96° 20,8 g Métabisulfite de sodium en solution aqueuse à 35% 0,23 g M.A Sel pentasodique de l'acide diéthylène-triamine-pentaacétique en solution aqueuse à 40% 0,48 g M.A Alkyl en C₈-C₁₀ polyglucoside en solution aqueuse à 60% 3,6 g M.A Alcool benzylique 2,0 g Polyéthylène glycol à 8 motifs d'oxyde d'éthylène 3,0 g NH₄Cl 4,32 g Ammoniaque à 20% de NH₃ 2,94 g | | | | | |

Au moment de l'emploi, chaque composition est mélangée avec un poids égal d'eau oxygénée à 20 volumes (6% en poids). On obtient un pH final de 9,5.
Chaque mélange obtenu est appliqué sur des mèches de cheveux gris à 90 % de blancs. Après 30 minutes de pose, les mèches sont rincées, lavées avec un shampooing standard, rincées à nouveau puis séchées.
Les nuances obtenues figurent dans le tableau ci-dessous :

| **Exemple** | **7** | **8** | **9** | **10** | **11** |
|---|---|---|---|---|---|
| **Nuance observée** | rouge | orangé | rouge | rouge chromatique | bleu intense |

### Exemples 12 à 24 : Composition tinctoriale à partir du 2-[(4-aminophényl)amino]-N(tétrahydrofuran-2-ylméthyl)éthanesulfonamide, dichlorhydrate (11)

### -Exemples 12 à 18 : Teinture en milieu acide

Les compositions tinctoriales suivantes sont préparées :

| **Exemple** | **12** | **13** | **14** | **15** | **16** | **17** | **18** |
|---|---|---|---|---|---|---|---|
| 2-[(4-aminophenyl)amino]-N(tetrahydrofuran-2-ylmethyl) ethanesulfonamide dichlorhydrate (11) | 10-3 mole | 10-3 mole | 10-3 mole | 10-3 mole | 10-3 mole | 10-3 mole | 10-3 mole |
| Benzene-1,3-diol | 10-3 mole | | | | | | |
| 5-Amino-2-methyl-phenol | | 10-3 mole | | | | | |
| 1H-Indol-6-ol | | | 10-3 mole | | | | |
| 2-Amino-pyridin-3-ol | | | | 10-3 mole | | | |
| 3,6-Dimethyl-1H-pyrazolo[5,1-c][1,2 ,4]triazole | | | | | ₁₀₋₃ mole | | |
| 2-(2,4-Diamino-phenoxy)- | | | | | | 10-3 mole | |
| 3-Amino-2-chloro-6-methyl- | | | | | | | 10-3 mole |
| Support de teinture (1) | (*) | (*) | (*) | (*) | (*) | (*) | (*) |
| Eau déminéralisée | 100g | 100g | 100g | 100g | 100g | 100g | 100g |

| | | | | | | | |
|---|---|---|---|---|---|---|---|
| (*) : support de teinture (1) pH 7 Alcool éthylique à 96° 20,8 g Métabisulfite de sodium en solution aqueuse à 35% 0,23 g M.A Sel pentasodique de l'acide diéthylène-triamine-pentaacétique en solution aqueuse à 40% 0,48 gM.A Alkyl en C₈-C₁₀ polyglucoside en solution aqueuse à 60% 3,6 g M.A Alcool benzylique 2,0 g Polyéthylène glycol à 8 motifs d'oxyde d'éthylène 3,0 g Na₂HPO₄ 0,28 g KH₂PO₄ 0,46 g | | | | | | | |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.
Les nuances obtenues figurent dans le tableau ci-dessous:

| **Exemple** | **12** | **13** | **14** | **15** | **16** | **17** | **18** |
|---|---|---|---|---|---|---|---|
| **Nuance observée** | brun intense | violet-rouge intense | brun rouge intense | brun rouge intense | gris rouge intense | bleu intense | violet intense |

### - Exemples 19 à 24 : teinture en milieu basique

Les compositions tinctoriales suivantes sont préparées :

| **Exemple** | **19** | **20** | **21** | **22** | **23** | **24** |
|---|---|---|---|---|---|---|
| 2-[(4-aminophenyl)amino]-N(tetrahydrofuran-2-ylmethyl) ethanesulfonamide dichlorhydrate (11) | 10-3 mole | 10-3 mole | 10-3 mole | 10-3 mole | 10-3 mole | 10-3 mole |
| 5-Amino-2-methyl-phenol | 10-3 mole | | | | | |
| 1H-Indol-6-ol | | 10-3 mole | | | | |
| 2-Amino-pyridin-3-ol | | | 10-3 mole | | | |
| 3, 6-Dimethyl-1 H-pyrazolo[5,1-c][1,2 ,4]triazole | | | | 10-3 mole | | |
| 2-(2,4-Diamino-phenoxy)-ethanol,chlorhydrate | | | | | 10-3 mole | |
| 3-Amino-2-chloro-6-methyl-phenol,chlorhydrate | | | | | | 10-3 mole 10-3 mole |
| Support de teinture (2) | (*) | (*) | (*) | (*) | (*) | (*) |
| Eau déminéralisée q.s.p. | 100g | 100g | 100g | 100g | 100g | 100g |

| | | | | | | |
|---|---|---|---|---|---|---|
| (*) : support de teinture (2) pH 9.5 Alcool éthylique à 96° 20,8 g Métabisulfite de sodium en solution aqueuse à 35% 0,23 g M.A Sel pentasodique de l'acide diéthylène-triamine-pentaacétique en solution aqueuse à 40% 0,48 g M.A Alkyl en C₈-C₁₀ polyglucoside en solution aqueuse à 60% 3,6 g M.A Alcool benzylique 2,0 g Polyéthylène glycol à 8 motifs d'oxyde d'éthylène 3,0 g NH₄Cl 4,32 g Ammoniaque à 20% de NH₃ 2,94 g | | | | | | |

Au moment de l'emploi, chaque composition est mélangée avec un poids égal d'eau oxygénée à 20 volumes (6% en poids). On obtient un pH final de 9,5.
Chaque mélange obtenu est appliqué sur des mèches de cheveux gris à 90 % de blancs. Après 30 minutes de pose, les mèches sont rincées, lavées avec un shampooing standard, rincées à nouveau puis séchées.
Les nuances obtenues figurent dans le tableau ci-dessous :

| **Exemple** | **19** | **20** | **21** | **22** | **23** | **24** |
|---|---|---|---|---|---|---|
| **Nuance observée** | rouge | orangé | rouge | rouge chromatique | bleu intense | violet intense |

### Exemples 25 à 29: Composition tinctoriale à partir du 2-(4-Amino-phénylamino)-éthanesulfonamide (13) -Exemples 25 à 28 : Teinture en milieu acide

Les compositions tinctoriales suivantes sont préparées :

| **Exemple** | **25** | **26** | **27** | **28** |
|---|---|---|---|---|
| 2-(4-Amino-phenylamino)- 10-3 mole 10-3 mole ethanesulfonamide (13) | | | 10-3 mole | 10-3 mole |
| 5-Amino-2-methyl-phenol | 10-3 mole | | | |
| 1H-Indol-6-ol | | 10-3 mole | | |
| 2-Amino-pyridin-3-ol | | | 10-3 mole | |
| 2-(2,4-Diamino-phenoxy)-ethanol, chlorhydrate | | | | 10-3 mole |
| Support de teinture (1) | (*) | (*) | (*) | (*) |
| Eau déminéralisée q.s.p. | 100g | 100g | 100g | 100g |

| | | | | |
|---|---|---|---|---|
| (*) : support de teinture (1) pH 7 Alcool éthylique à 96° 20,8 g Métabisulfite de sodium en solution aqueuse à 35% 0,23 g M.A Sel pentasodique de l'acide diéthylène-triamine-pentaacétique en solution aqueuse à 40% 0,48 g M.A Alkyl en C8-C10 polyglucoside en solution aqueuse à 60% 3,6 g M.A Alcool benzylique 2,0 g Polyéthylène glycol à 8 motifs d'oxyde d'éthylène 3,0 g Na₂HPO₄ 0,28 g KH₂PO₄ 0,46 g | | | | |

Au moment de l'emploi, chaque composition est mélangée avec un poids égal d'eau oxygénée à 20 volumes (6% en poids). On obtient un pH final de 7.
Chaque mélange obtenu est appliqué sur des mèches de cheveux gris à 90 % de blancs. Après 30 minutes de pose, les mèches sont rincées, lavées avec un shampooing standard, rincées à nouveau puis séchées.
Les nuances obtenues figurent dans le tableau ci-dessous:

| **Exemple** | **25** | **26** | **27** | **28** |
|---|---|---|---|---|
| **Nuance observée** | rouge | brun orangé | brun rouge | gris bleu intense |

### - Exemples 29 : teinture en milieu basique

La composition tinctoriale suivante est préparée :

| **Exemple** | **29** |
|---|---|
| 2-(4-Amino-phenylamino)-ethanesulfonamide (13) | 10-3 mole |
| 2-(2,4-Diamino-phenoxy)-ethanol,chlorhydrate | 10-3 mole |
| Support de teinture (2) | (*) |
| Eau déminéralisée q.s.p. | 100g |

| | |
|---|---|
| (*) : support de teinture (2) pH 9.5 Alcool éthylique à 96° 20,8 g Métabisulfite de sodium en solution aqueuse à 35% 0,23 g M.A Sel pentasodique de l'acide diéthylène-triamine-pentaacétique en solution aqueuse à 40% 0,48 g M.A Alkyl en C₈-C₁₀ polyglucoside en solution aqueuse à 60% 3,6 g M.A Alcool benzylique 2,0 g Polyéthylène glycol à 8 motifs d'oxyde d'éthylène 3,0 g NH₄Cl 4,32 g Ammoniaque à 20% de NH₃ 2,94 g | |

Au moment de l'emploi, la composition est mélangée avec un poids égal d'eau oxygénée à 20 volumes (6% en poids). On obtient un pH final de 9,5.

Le mélange obtenu est appliqué sur des mèches de cheveux gris à 90 % de blancs. Après 30 minutes de pose, les mèches sont rincées, lavées avec un shampooing standard, rincées à nouveau puis séchées.
La nuance obtenue figure dans le tableau ci-dessous:

| **Exemple** | **29** |
|---|---|
| **Nuance observée** | gris bleu |

### Exemples 30 à 40 : Composition tinctoriale à partir du 2-(4-Amino-phénylamino)-éthanesulfonyl méthylamide (15)

### -Exemples 30 à 35 : Teinture en milieu acide

Les compositions tinctoriales suivantes sont préparées :

| **Exemple** | **30** | **31** | **32** | **33** | **34** | **35** |
|---|---|---|---|---|---|---|
| 2-(4-Amino-phenylamino)-ethanesulfonyl methylamide (15) | 10-3 mole | 10-3 mole | 10-3 mole | 10-3 mole | 10-3 mole | 10-3 mole |
| Benzene-1,3-diol | 10-3 mole | | | | | |
| 5-Amino-2-methyl-phenol | | 10-3 mole | | | | |
| 1H-Indol-6-ol | | | 10-3 mole | | | |
| 2-Amino-pyridin-3-ol | | | | 10-3 mole | | |
| 3,6-Dimethyl-1H-pyrazolo[5,1-c][1,2 ,4]triazole | | | | | 10-3 mole | |
| 2-(2,4-Diamino-phenoxy)-ethanol,chlorhydrate | | | | | | 10-3 mole |
| Support de teinture (1) | (*) | (*) | (*) | (*) | (*) | (*) |
| Eau déminéralisée q.s.p. | 100g | 104g | 100g | 100g | 100g | 100g |

| | | | | | | |
|---|---|---|---|---|---|---|
| (*) : support de teinture (1) pH 7 Alcool éthylique à 96° 20,8 g Métabisulfite de sodium en solution aqueuse à 35% 0,23 g M.A Sel pentasodique de l'acide diéthylène-triamine-pentaacétique en solution aqueuse à 40% 0,48 g M.A Alkyl en C8-C10 polyglucoside en solution aqueuse à 60% 3,6 g M.A Alcool benzylique 2,0 g Polyéthylène glycol à 8 motifs d'oxyde d'éthylène 3,0 g Na₂HPO₄ 0,28 g KH₂PO₄ 0,46 g | | | | | | |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pause, les mèches sont rincées, lavées avec un shampooing standard, rincées à nouveau puis séchées.
Les nuances obtenues figurent dans le tableau ci-dessous :

| **Exemple** | **30** | **31** | **32** | **33** | **34** | **35** |
|---|---|---|---|---|---|---|
| **Nuance observée** | brun intense | violet-rouge intense | brun rouge intense | rouge intense | brun intense | violet-bleu intense |

### - Exemples 36 à 40 : teinture en milieu basique

Les compositions tinctoriales suivantes sont préparées :

| **Exemple** | **36** | **37** | **38** | **39** | **40** |
|---|---|---|---|---|---|
| 2-(4-Amino-phenylamino)-ethanesulfonyl methylamide (15) | 10-3 mole | 10-3 mole | 10-3 mole | 10-3 mole | 10-3 mole |
| 5-Amino-2-methyl-phenol | 10-3 mole | | | | |
| 1H-Indol-6-ol | | 10-3 mole | | | |
| 2-Amino-pyridin-3-ol | | | 10-3 mole | | |
| 3,6-Dimethyl-1H-pyrazolo[5,1-c][1,2 4]triazole | | | | 10-3 mole | |
| 2-(2,4-Diamino-phenoxy)-ethanol,chlorhydrate | | | | | 10-3 mole |
| Support de teinture (2) | (*) | (*) | (*) | (*) | (*) |
| Eau déminéralisée q.s.p. | 100g | 100g | 100g | 100g | 100g |

| | | | | | |
|---|---|---|---|---|---|
| (*) : support de teinture (2) pH 9.5 Alcool éthylique à 96° 20,8 g Métabisulfite de sodium en solution aqueuse à 35% 0,23 g M.A Sel pentasodique de l'acide diéthylène-triamine-pentaacétique en solution aqueuse à 40% 0,48 g M.A Alkyl en C₈-C₁₀ polyglucoside en solution aqueuse à 60% 3,6 g M.A Alcool benzylique 2,0 g Polyéthylène glycol à 8 motifs d'oxyde d'éthylène 3,0 g NH₄Cl 4,32 g Ammoniaque à 20% de NH₃ 2,94 g | | | | | |

Au moment de l'emploi, chaque composition est mélangée avec un poids égal d'eau oxygénée à 20 volumes (6% en poids). On obtient un pH final de 9,5.
Chaque mélange obtenu est appliqué sur des mèches de cheveux gris à 90 % de blancs. Après 30 minutes de pose, les mèches sont rincées, lavées avec un shampooing standard, rincées à nouveau puis séchées.
Les nuances obtenues figurent dans le tableau ci-dessous :

| **Exemple** | **36** | **37** | **38** | **39** | **40** |
|---|---|---|---|---|---|
| **Nuance observée** | rouge | orangé | rouge | rouge chromatique | bleu intense |

### Exemples 41 à 51 : Composition tinctoriale à partir du 2-(4-amino-phénylamino)-éthanesulfonyl (2-hydroxy-éthyl)-amide (17)

### -Exemples 41 à 47 : Teinture en milieu acide

Les compositions tinctoriales suivantes sont préparées :

| **Exemple** | **41** | **42** | **43** | **44** | **45** | **46** | **47** |
|---|---|---|---|---|---|---|---|
| acide 2-(4-amino-phenylamino)-ethanesulfonique (2-hydroxy-ethyl)-amide (17) | 10-3 mole | 10-3 mole | 10-3 mole | 10-3 mole | 10-3 mole | 10-3 mole | 10-3 mole |
| Benzene-1,3-diol | 10-3 mole | | | | | | |
| 5-Amino-2-methyl-phenol | | 10-3 mole | | | | | |
| 1H-Indol-6-ol | | | 10-3 mole | | | | |
| 2 Amino-pyridin-3-ol | | | | 10-3 mole | | | |
| 3,6-Dimethyl-1H-pyrazolo[5,1-c][1,2 4]triazole | | | | | 10-3 mole | | |
| 2-(2,4-Diamino-phenoxy)-ethanol,chlorhydrate | | | | | | 10-3 mole | |
| 3-Amino-2-chloro-6-methyl-phenol, chlorhydrate | | | | | | | 10-3 mole |
| Support de teinture (1) | (*) | (*) | (*) | (*) | (*) | (*) | (*) |
| Eau déminéralisée q.s.p. | 100g | 100g | 100q | 100q | 100g | 100g | 100g |

| | | | | | | | |
|---|---|---|---|---|---|---|---|
| (*) : support de teinture (1) pH 7 Alcool éthylique à 96° 20,8 g Métabisulfite de sodium en solution aqueuse à 35% 0,23 g M.A Sel pentasodique de l'acide diéthylène-triamine-pentaacétique en solution aqueuse à 40% 0,48 g M.A Alkyle en C8-C10 polyglucoside en solution aqueuse à 60% 3,6 g M.A Alcool benzylique 2,0 g Polyéthylène glycol à 8 motifs d'oxyde d'éthylène 3,0 g Na2HPO4 0,28 g KH2PO4 0,46 g | | | | | | | |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.
Les nuances obtenues figurent dans le tableau ci-dessous :

| **Exemple** | **41** | **42** | **43** | **44** | **45** | **46** | **47** |
|---|---|---|---|---|---|---|---|
| **Nuance observée** | brun orangé | | brun rouge | rouge rouge | brun orangé | gris bleu intense | violet intense |

### Exemples 48 à 51: teinture en milieu basique

Les compositions tinctoriales suivantes sont préparées :

| **Exemple** | **48** | **49** | **50** | **51** |
|---|---|---|---|---|
| acide 2-(4-amino-phenylamino)-ethanesulfonique (2-hydroxy-ethyl)-amide | 10-3 mole | 10-3 mole | 10-3 mole | 10-3 mole |
| 1H-Indol-6-ol | 10-3 mole | | | |
| 3,6-Dimethyl-1H-pyrazolo[5,1-c][1,2 ,4]triazole | | 10-3 mole | | |
| 2-(2,4-Diamino-phenoxy)- | | | 10-3 mole | |
| 3-Amino-2-chloro-6-methyl- | | | | 10-3 mole |
| Support de teinture (2) | (*) | (*) | (*) | (*) |
| Eau déminéralisée q.s.p. | 100q | 100q | 100q | 100q |

| | | | | |
|---|---|---|---|---|
| (*) : support de teinture (2) pH 9.5 Alcool éthylique à 96° 20,8 g Métabisulfite de sodium en solution aqueuse à 35% 0,23 g M.A Sel pentasodique de l'acide diéthylène-triamine- pentaacétique en solution aqueuse à 40% 0,48 g M.A Alkyl en C₈-C₁₀ polyglucoside en solution aqueuse à 60% 3,6 g M.A Alcool benzylique 2,0 g Polyéthylène glycol à 8 motifs d'oxyde d'éthylène 3,0 g NH₄Cl 4,32 g Ammoniaque à 20% de NH₃ 2,94 g | | | | |

Au moment de l'emploi, chaque composition est mélangée avec un poids égal d'eau oxygénée à 20 volumes (6% en poids). On obtient un pH final de 9,5.
Chaque mélange obtenu est appliqué sur des mèches de cheveux gris à 90 % de blancs. Après 30 minutes de pose, les mèches sont rincées, lavées avec un shampooing standard, rincées à nouveau puis séchées.
Les nuances obtenues figurent dans le tableau ci-dessous :

| **Exemple** | **48** | **49** | **50** | **51** |
|---|---|---|---|---|
| **Nuance observée** | orangé | rouge | bleu | violet |

### Exemples 52 à 65 : Composition tinctoriale à partir du N-[2-(4-Amino-phénylamino)-éthyl]-N-méthyl-méthanesulfonamide, sulfate (24)

### - Exemples 52 à 58 : Teinture en milieu acide

Les compositions tinctoriales suivantes sont préparées :

| **Exemple** | **52** | **53** | **54** | **55** | **56** | **57** | **58** |
|---|---|---|---|---|---|---|---|
| ethyl]-N-methyl-methanesulfonamide, sulfate | 10-3 mole | 10-3 mole | 10-3 mole | 10-3 mole | 10-3 mole | 10-3 mole | 10-3 mole |
| Benzene-1,3-diol | 10-3 mole | | | | | | |
| 5-Amino-2-methyl-phenol | | mole mole | | | | | |
| 1H-Indol-6-ol | | | 10-3 mole | | | | |
| 2-Amino-pyridin-3-ol | | | | | | | |
| 3,6-Dimethyl-1 H-pyrazolo[5,1-c][1.2.4]triazole | | | | | 10-3 mole | | |
| 2-(2,4-Diamino-phenoxy)-ethanol, chlorhydrate | | | | | | 10-3 mole | |
| 3-Amino-2-chloro-6-methyl-phenol, chlorhydrate | | | | | | | 10-3 mole |
| Support de teinture (1) | (*) | (*) | (*) | (*) | (*) | (*) | (*) |
| Eau déminéralisée q.s.p. | 100g | 100g | 100g | 100g | 100g | 100g | 100g |

| | | | | | | | |
|---|---|---|---|---|---|---|---|
| (*) : support de teinture (1) pH 7 Alcool éthylique à 96° 20,8 g Métabisulfite de sodium en solution aqueuse à 35% 0,23 g M.A Sel pentasodique de l'acide diéthylène-triamine-pentaacétique en solution aqueuse à 40% 0,48 g M.A Alkyl en C₈-C₁₀ polyglucoside en solution aqueuse à 60% 3,6 g M.A Alcool benzylique 2,0 g Polyéthylène glycol à 8 motifs d'oxyde d'éthylène 3,0 g Na₂HPO₄ 0,28 g KH₂PO₄ 0,46 g | | | | | | | |

Au moment de l'emploi, chaque composition est mélangée avec un poids égal d'eau oxygénée à 20 volumes (6% en poids). On obtient un pH final de 7.
Chaque mélange obtenu est appliqué sur des mèches de cheveux gris à 90 % de blancs. Après 30 minutes de pose, les mèches sont rincées, lavées avec un shampooing standard, rincées à nouveau puis séchées.
Les nuances obtenues figurent dans le tableau ci-dessous :

| **Exemple** | **52** | **53** | **54** | **55** | **56** | **57** | **58** |
|---|---|---|---|---|---|---|---|
| Nuance observée | gris intense | gris violet intense | brun rouge intense | brun rouge intense | gris intense | bleu intense | violet intense |

### - Exemples 59 à 65 : teinture en milieu basique

Les compositions tinctoriales suivantes sont préparées :

| Exemple | 59 | 60 | 61 | 62 | 63 | 64 | 65 |
|---|---|---|---|---|---|---|---|
| N-[2-(4-Amino-phenylamino)-ethyl]-N-methyl-methanesulfonamide, sulfate (24) | 10-3 mole | 10-3 mole | 10-3 mole | 10-3 mole | 10-3 mole | 10-3 mole | 10-3 mole |
| Benzene-1,3-diol | 10-3 mole | | | | | | |
| 5-Amino-2-methyl-phenol | | 10-3 mole | | | | | |
| 1H-Indol-6-ol | | | 10-3 mole | | | | |
| 2-Amino-pyridin-3-ol | | | | 10-3 mote | | | |
| 3,6-Dimethyl-1 H-pyrazolo[5,1-c][1,2 ,4]triazole | | | | | 10-3 mole | | |
| 2-(2,4-Diamino-phenoxy)-ethanol.chlorhydrate | | | | | | 10-3 mole | |
| 3-Amino-2-chloro-6-methyl-phenol,chlorhydrate | | | | | | | 10-3 mole 10-3 mole |
| Support de teinture (2) | (*) | (*) | (*) | (*) | (*) | (*) | (*) |
| Eau déminéralisée q.s.p. | 100q | 100q | 100q | 100q | 100q | 100q | 100q |

| | | | | | | | |
|---|---|---|---|---|---|---|---|
| (*) : support de teinture (2) pH 9.5 Alcool éthylique à 96° 20,8 g Métabisulfite de sodium en solution aqueuse à 35% 0,23 g M.A Sel pentasodique de l'acide diéthylène-triamine-pentaacétique en solution aqueuse à 40% 0,48 g M.A Alkyl en C₈-C₁₀ polyglucoside en solution aqueuse à 60% 3,6 g M.A Alcool benzylique 2,0 g Polyéthylène glycol à 8 motifs d'oxyde d'éthylène 3,0 g NH₄Cl 4,32 g Ammoniaque à 20% de NH₃ 2,94 g | | | | | | | |

Au moment de l'emploi, chaque composition est mélangée avec un poids égal d'eau oxygénée à 20 volumes (6% en poids). On obtient un pH final de 9,5.
Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| **Exemple** | **59** | **60** | **61** | **62** | **63** | **64** | **65** |
|---|---|---|---|---|---|---|---|
| **Nuance observée** | jaune | violet-rouge | rouge | rouge | rouge chromatiq | bleu intense | violet intense |

### Exemples 66 à 77 : Composition tinctoriale à partir du N-[4-(4-Amino-phénylamino)-butyl]-méthanesulfonamide (27)

### -Exemples 66 à 71 : Teinture en milieu acide

Les compositions tinctoriales suivantes sont préparées :

| **Exemple** | **66** | **67** | **68** | **69** | **70** | **71** |
|---|---|---|---|---|---|---|
| N-[4-(4-Amino-phenylamino)-butyl]-methanesulfonamide, sulfate (27) | 10-3 mole | 10-3 mole | 10-3 mole | 10-3 mole | 10-3 mole | 10-3 mole |
| Benzene-1,3-diol | 10-3 mole | | | | | |
| 5-Amino-2-methyl-phenol | | 10-3 mole | | | | |
| 1H-Indol-6-ol | | | 10-3 mole | | | |
| 2-Amino-pyridin-3-ol | | | | 10-3 mole | | |
| 3,6-Dimethyl-1H-pyrazolo[5,1-c][1,2,4]triazole | | | | | 10-3 mole | |
| 2-(2,4-Diamino-phenoxy)-ethanol,chlorhydrate | | | | | | 10-3 mole |
| Support de teinture (1) | (*) | (*) | (*) | (*) | (*) | (*) |
| Eau déminéralisée q.s.p. | 100g | 100g | 100g | 100g | 100g | 100g |

| | | | | | | |
|---|---|---|---|---|---|---|
| (*) : support de teinture (1) pH 7 Alcool éthylique à 96° 20,8 g Métabisulfite de sodium en solution aqueuse à 35% 0,23 g M.A Sel pentasodique de l'acide diéthylène-triamine-pentaacétique en solution aqueuse à 40% 0,48 g M.A Alkyl en C₈-C₁₀ polyglucoside en solution aqueuse à 60% 3,6 g M.A Alcool benzylique 2,0 g Polyéthylène glycol à 8 motifs d'oxyde d'éthylène 3,0 g Na₂HPO₄ 0,28 g KH₂PO₄ 0,46 g | | | | | | |

Au moment de l'emploi, chaque composition est mélangée avec un poids égal d'eau oxygénée à 20 volumes (6% en poids). On obtient un pH final de 7.
Chaque mélange obtenu est appliqué sur des mèches de cheveux gris à 90 % de blancs. Après 30 minutes de pose, les mèches sont rincées, lavées avec un shampooing standard, rincées à nouveau puis séchées.
Les nuances obtenues figurent dans le tableau ci-dessous :

| **Exemple** | **66** | **67** | **68** | **69** | **70** | **71** |
|---|---|---|---|---|---|---|
| **Nuance observée** | brun intense | violet | gris | rouge intense intense | violet- gris rouge intense | bleu intense |

### - Exemples 72 à 77 : teinture en milieu basique

Les compositions tinctoriales suivantes sont préparées :

| **Exemple** | **72** | **73** | **74** | **75** | **76** | **77** |
|---|---|---|---|---|---|---|
| N-[4-(4-Amino-phenylamino)-butyl]-methanesulfonamide, sulfate (27) | 10-3 mole | 10-3 mole | 10-3 mole | 10-3 mole | 10-3 mole | 10-3 mole |
| Benzene-1,3-diol | 10-3 mole | | | | | |
| 5-Amino-2-methyl-phenol | | 10-3 mole | | | | |
| 1H-Indol-6-ol | | | 10-3 mole | | | |
| 2-Amino-pyridin-3-ol | | | | 10-3 mole | | |
| 3,6-Dimethyl-1 H-pyrazolo[5,1-c][1,2,4]triazole | | | | | 10-3 mole | |
| 2-(2,4-Diamino-phenoxy)-ethanol,chlorhydrate | | | | | | 10-3 mole |
| Support de teinture (2) | (*) | (*) | (*) | (*) | (*) | (*) |
| Eau déminéralisée q.s.p. | 100g | 100g | 100g | 100g | 100g | 100g |

| | | | | | | |
|---|---|---|---|---|---|---|
| (*) : support de teinture (2) pH 9.5 Alcool éthylique à 96° 20,8 g Métabisulfite de sodium en solution aqueuse à 35% 0,23 g M.A Sel pentasodique de l'acide diéthylène-triamine-pentaacétique en solution aqueuse à 40% 0,48 g M.A Alkyl en C₈-C₁₀polyglucoside en solution aqueuse à 60% 3,6 g M.A Alcool benzylique 2,0 g Polyéthylène glycol à 8 motifs d'oxyde d'éthylène 3,0 g NH₄Cl 4,32 g Ammoniaque à 20% de NH₃ 2,94 g | | | | | | |

Au moment de l'emploi, chaque composition est mélangée avec un poids égal d'eau oxygénée à 20 volumes (6% en poids). On obtient un pH final de 9,5.
Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous:

| **Exemple** | **72** | **73** | **74** | **75** | **76** | **77** |
|---|---|---|---|---|---|---|
| **Nuance observée** | jaune | violet intense | rouge | brun rouge | rouge intense | bleu intense |

### Exemples 78 à 82 : Composition tinctoriale à partir de l'acide 2-(4-Amino-phenylamino)-4-methylsulfanyl-butyric methyl ester, dichlorhydrate (29)

### - Exemples 78 à 82 : Teinture en milieu acide

Les compositions tinctoriales suivantes sont préparées :

| **Exemple** | **78** | **79** | **80** | **81** | **82** |
|---|---|---|---|---|---|
| 2-(4-Amino-phenylamino)-4-methylsulfanyl-butyric acid methyl ester, dichlorhydrate (29) | 10-3 mole | 10-3 mole | 10-3 mole | 10-3 mole | 10-3 mole |
| 5-Amino-2-methyl-phenol | 10-3 mole | | | | |
| 1H-hdol-6-ol | | 10-3 mole | | | |
| 2-Amino-pyridin-3-ol | | | 10-3 mole | | |
| 2-(2,4-Diamino-phenoxy)-ethanol,chlorhydrate | | | | 10-3 mole | |
| 3-Amino-2-chloro-6-methyl-phenol,chlorhydrate | | | | | 10-3 mole |
| Support de teinture (1) | (*) | (*) | (*) | (*) | (*) |
| Eau déminéralisée q.s.p. | 100g | 100g | 100g | 100g | 100g |

| | | | | | |
|---|---|---|---|---|---|
| (*) : support de teinture (1) pH 7 Alcool éthylique à 96° 20,8 g Métabisulfite de sodium en solution aqueuse à 35% 0,23 g M.A Sel pentasodique de l'acide diéthylène-triamine-pentaacétique en solution aqueuse à 40% 0,48 g M.A Alkyl en C₈-C₁₀ polyglucoside en solution aqueuse à 60% 3,6 g M.A Alcool benzylique 2,0 g Polyéthylène glycol à 8 motifs d'oxyde d'éthylène 3,0 g Na₂HPO₄ 0,28 g KH₂PO₄ 0,46 g | | | | | |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| **Exemple** | **78** | **79** | **80** | **81** | **82** |
|---|---|---|---|---|---|
| **Nuance observée** | rouge | brun | rouge | gris intense | rouge |

### - Exemples 83 à 84: teinture en milieu basique

Les compositions tinctoriales suivantes sont préparées :

| **Exemple** | **83** | **84** |
|---|---|---|
| 2-(4-Amino-phenylamino)-4-methylsulfanyl-butyric acid methyl ester, dichlorhydrate (29) | 10-3 mole | 10-3 mole |
| 2-(2,4-Diamino-phenoxy)-ethanol,chlorhydrate | 10-3 mole | |
| 3-Amino-2-chloro-6-methyl-phenol,chlorhydrate | | 10-3 mole |
| Support de teinture (2) | (*) | (*) |
| Eau déminéralisée q.s.p. | 100g | 100g |

| | | |
|---|---|---|
| (*) : support de teinture (2) pH 9.5 Alcool éthylique à 96° 20,8 g Métabisulfite de sodium en solution aqueuse à 35% 0,23 g M.A Sel pentasodique de l'acide diéthylène-triamine-pentaacétique en solution aqueuse à 40% 0,48 g M.A Alkyl en C₈-C₁₀ polyglucoside en solution aqueuse à 60% 3,6 g M.A Alcool benzylique 2,0 g Polyéthylène glycol à 8 motifs d'oxyde d'éthylène 3,0 g NH₄Cl 4,32 g Ammoniaque à 20% de NH₃ 2,94 g | | |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| **Exemple** | **83** | **84** |
|---|---|---|
| **Nuance observée** | gris violet-bleu | rouge |

## Revendications

1. Composés **caractérisé en ce qu'**il s'agit d'une para-phénylènediamine secondaire soufrée de formule générale (I) : 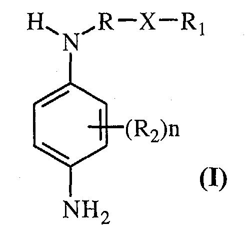 dans laquelle :
le groupement X représente un radical SO₂ ou un atome de soufre,
si X représente un radical SO₂, le groupement R représente un radical alkylène en C₂-C₁₀ linéaire ou ramifié, saturé ou insaturé, non-substitué ou substitué par un atome d'halogène ou par un ou plusieurs groupements hydroxy, alcoxy, amino, mono- ou di-alkylamino, alkylcarbonyle, carboxy, amido, alcoxycarbonyle ou mono- ou di-alkylamino carbonyle ; un ou plusieurs atomes de carbone de ce radical alkylène pouvant être remplacés par un ou plusieurs hétéroatomes choisi parmi l'oxygène, l'azote et le soufre, ou par une ou plusieurs fonctions carbonyles,
si X représente un atome de soufre, le groupement R représente un radical alkylène en C₁-C₁₀ ramifié, saturé ou insaturé, non-substitué ou substitué par un atome d'halogène ou par un ou plusieurs groupements hydroxy, alcoxy, amino, mono- ou di-alkylamino, alkylcarbonyle, carboxy, amido, alcoxycarbonyle ou mono- ou di-alkylamino carbonyle ; un ou plusieurs atomes de carbones de ce radical alkylène pouvant être remplacés par un ou plusieurs hétéroatomes choisi parmi l'oxygène, l'azote et le soufre, ou par une ou plusieurs fonctions carbonyles,
le groupement R₁ représente un radical amino, un radical aryle ou un radical alkyle en C₁-C₁₀ linéaire ou ramifié, saturé ou insaturé, non-substitué ou substitué par un atome d'halogène ou par un ou plusieurs groupements hydroxy, alcoxy, amino, mono- ou di-alkylamino, alkylcarbonyle, carboxy, amido, alcoxycarbonyle, mono- ou di-alkylamino carbonyle ou un hétérocycle à 5 ou 6 atomes saturé ou insaturé ; un ou plusieurs atomes de carbone de ce radical alkyle pouvant être remplacés par un ou plusieurs hétéroatomes choisi parmi l'oxygène, l'azote et le soufre, ou par une ou plusieurs fonctions carbonyles,
le groupement R₂ représente un atome d'hydrogène ou d'halogène, un radical choisi parmi alkyle, alcoxy, alcoxy alkyle, hydroxy alcoxy ou mono- ou poly-hydroxy alkyle,
n représente un entier variant de 1 à 4,
ou ses sels d'addition
à l'exception des composés suivants :
- 2-(4-Amino-3-méthyl-phénylamino)-éthanesulfonamide,
- 2-(4-Amino-phénylamino)-éthanesulfonic acid diéthylamide,
- N-[2-(4-Amino-2-méthyl-phénylamino)-éthyl]-méthanesulfonamide,
- N-[2-(4-Amino-phénylamino)-éthyl]-méthanesulfonamide,
- 2-[3-(4-Amino-phénylamino)-propane-1-sulfonyl]-éthanol,
- N-[3-(Butane-1-sulfonyl)-propyl]-benzène-1,4-diamine,
- N-[2-(4-Amino-3-méthyl-phénylamino)-éthyl]-méthanesulfonamide,
- N-[2-(4-Amino-2-méthyl-phénylamino)-éthyl]-N-éthyl-méthanesulfonamide,
- Acide [2-(4-Amino-3-méthyl-phénylamino)-éthyl]-sulfamique,
- N-[2-(4-Amino-2-chloro-phénylamino)-éthyl]-méthanesulfonamide,
- N-[3-(4-Amino-phénylamino)-propyl-3-oxo]-benzènesulfonamide.

2. Composé selon la revendication 1, **caractérisé en ce que** le groupement R de la formule (I) représente un alkylène linéaire ou ramifié en C₂-C₅, pouvant être substitué par un radical choisi parmi -OH, -COOCH₃ et -COOCH₂CH₃, un ou plusieurs atomes de carbone de ce radical alkylène pouvant être remplacés par un ou plusieurs atomes d'oxygène, d'azote ou de soufre.

3. Composé selon l'une des revendications 1 ou 2, **caractérisé en ce que** le groupement R₁ de la formule (I) représente un groupement alkyle, aryle, arylalkyle, hydroxyalkyle, amine secondaire ou tertiaire.

4. Composé selon l'une quelconque des revendications 1 à 3, **caractérisé en ce que** le groupement R₂ de la formule (I) représente un atome d'hydrogène ou un groupement méthyle.

5. Composé selon l'une quelconque des revendications 1 à 4, **caractérisé en ce que** le composé de formule (I) est choisi parmi les composés suivants :
- N-[2-(4-amino-phénylamino)-éthyl]-benzènesulfonamide,
- Acide 2-(4-Amino-phénylamino)-éthanesulfonique (tetrahydro-furan-2-ylméthyl)-amide,
- Acide 2-(4-Amino-phénylamino)-éthanesulfonique méthylamide,
- Acide 2-(4-Amino-phénylamino)-éthanesulfonique (2-hydroxy-éthyl)-amide,
- Acide 2-(4-Amino-phénylamino)-éthanesulfonique amide,
- N-[2-(4-Amino-phénylamino)-éthyl]-N-méthyl-méthanesulfonamide,
- N-[4-(4-Amino-phénylamino)-butyl]-méthanesulfonamide,
- Acide 2-(4-amino-phénylamino)-éthanesulfonique bis-(2-hydroxy-éthyl)-amide,
- N-(2-Méthanesulfonyl-éthyl)-benzène-1,4-diamine,
- Acide méthanéthiosulfonique S-[2-(4-amino-phénylamino)-éthyl] ester,
- Acide 2-(4-amino-phénylamino)-4-méthylsulfanyl-butyrique méthyl ester,
- 2-(4-Amino-phénylamino)-3-benzylsulfanyl-propan-1-ol,
- Acide 2-(4-amino-phénylamino)-3-benzylsulfanyl-propionique éthyl ester,
- Acide 2-(4-amino-phénylamino)-3-benzylsulfanyl-propionique méthyl ester,
- Acide 2-(4-amino-phénylamino)-4-méthylsulfanyl-butyrique éthyl ester,
- N-[2-(4-Amino-2-méthyl-phénylamino)-éthyl]-benzènesulfonamide,
- Acide 2-(4-Amino-2-méthyl-phénylamino)-éthanesulfonique (tetrahydro-furan-2-ylméthyl)-amide,
- Acide 2-(4-Amino-2-méthyl-phénylamino)-éthanesulfonique méthylamide,
- Acide 2-(4-amino-2-méthyl-phénylamino)-éthanesulfonique (2-hydroxy-éthyl)-amide,
- Acide 2-(4-Amino-2-méthyl-phénylamino)-éthanesulfonique diéthylamide,
- Acide 2-(4-amino-2-méthyl-phénylamino)-éthanesulfonique amide,
- N-[2-(4-Amino-2-méthyl-phénylamino)-éthyl]-N-méthylméthane sulfonamide,
- N-[4-(4-Amino-2-méthyl-phénylamino)-butyl]-méthanesulfonamide,
- Acide 2-(4-amino-2-méthyl-phénylamino)-éthanesulfonique bis-(2-hydroxy-éthyl)-amide,
- N-1-(2-Méthanesulfonyl-éthyl)-2-méthyl-benzène-1,4-diamine,
- Acide méthanéthiosulfonique S-[2-(4-amino-2-méthyl-phénylamino)-éthyl] ester,
- Acide 2-(4-amino-2-méthyl-phénylamino)-4-méthylsulfanyl-butyrique méthyl ester,
- 2-(4-Amino-2-méthyl-phénylamino)-3-benzylsulfanyl-propan-1-ol,
- Acide 2-(4-amino-2-méthyl-phénylamino)-3-benzylsulfanyl-propionique éthyl ester,
- Acide 2-(4-Amino-2-méthyl-phénylamino)-3-benzylsulfanyl-propionique méthyl ester,
- N-[2-(4-Amino-3-méthyl-phénylamino)-éthyl]-benzènesulfonamide,
- Acide 2-(4-amino-3-méthyl-phénylamino)-éthanesulfonique (tetrahydro-furan-2-ylméthyl)-amide,
- Acide 2-(4-amino-3-méthyl-phénylamino)-éthanesulfonique méthylamide,
- Acide 2-(4-amino-3-méthyl-phénylamino)-éthanesulfonique (2-hydroxy-éthyl)-amide,
- Acide 2-(4-amino-3-méthyl-phénylamino)-4-méthylsulfanyl-butyrique éthyl ester,
- Acide 2-(4-amino-3-méthyl-phénylamino)-éthanesulfonique diéthylamide,
- Acide 2-(4-amino-2-méthyl-phénylamino)-4-méthylsulfanyl-butyrique éthyl ester,
- N-[2-(4-Amino-3-méthyl-phénylamino)-éthyl]-N-méthyl-méthane sulfonamide,
- N-[4-(4-Amino-3-méthyl-phénylamino)-butyl]-méthanesulfonamide,
- Acide 2-(4-Amino-3-méthyl-phénylamino)-éthanesulfonique bis-(2-hydroxy-éthyl)-amide,
- N-4-(2-Méthanesulfonyl-éthyl)-2-méthyl-benzène-1,4-diamine,
- Acide méthanéthiosulfonique S-[2-(4-amino-3-méthyl-phénylamino)-éthyl] ester,
- Acide 2-(4-amino-3-méthyl-phénylamino)-4-méthylsulfanyl-butyrique méthyl ester,
- 2-(4-Amino-3-méthyl-phénylamino)-3-benzylsulfanyl-propan-1-ol,
- Acide 2-(4-amino-3-méthyl-phénylamino)-3-benzylsulfanyl-propionique éthyl ester,
- Acide 2-(4-amino-3-méthyl-phénylamino)-3-benzylsulfanyl-propionique méthyl ester,
et leurs sels d'addition.

6. Composé selon l'une quelconque des revendications 1 à 5, **caractérisé en ce que** les sels d'addition avec un acide des composés de formule (I) sont choisis parmi les chlorhydrates, les bromhydrates, les sulfates, les citrates, les succinates, les tartrates, les lactates, les tosylates, les benzènesulfonates, les phosphates et les acétates.

7. Composé nitré **caractérisé en ce qu'**il possède la structure suivante (II) : 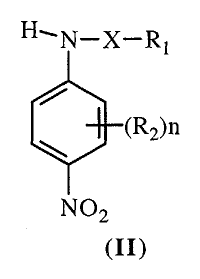 dans laquelle :
le groupement X représente un radical SO₂ ou un atome de soufre,
si X représente un radical SO₂, le groupement R représente un radical alkylène en C₂-C₁₀ linéaire ou ramifié, saturé ou insaturé, non-substitué ou substitué par un atome d'halogène ou par un ou plusieurs groupements hydroxy, alcoxy, amino, mono- ou di-alkylamino, alkylcarbonyle, carboxy, amido, alcoxycarbonyle ou mono- ou di-alkylamino carbonyle ; un ou plusieurs atomes de carbone de ce radical alkylène pouvant être remplacés par un ou plusieurs hétéroatomes choisi parmi l'oxygène, l'azote et le soufre, ou par une ou plusieurs fonctions carbonyles,
si X représente un atome de soufre, le groupement R représente un radical alkylène en C₁-C₁₀ ramifié, saturé ou insaturé, non-substitué ou substitué par un atome d'halogène ou par un ou plusieurs groupements hydroxy, alcoxy, amino, mono- ou di-alkylamino, alkylcarbonyle, carboxy, amido, alcoxycarbonyle ou mono- ou di-alkylamino carbonyle ; un ou plusieurs atomes de carbones de ce radical alkylène pouvant être remplacés par un ou plusieurs hétéroatomes choisi parmi l'oxygène, l'azote et le soufre, ou par une ou plusieurs fonctions carbonyles,
le groupement R₁ représente un radical amino, un radical -OY, dans lequel Y représente un atome d'hydrogène ou un métal, un radical aryle ou un radical alkyle en C₁-C₁₀ linéaire ou ramifié, saturé ou insaturé, non-substitué ou substitué par un atome d'halogène ou par un ou plusieurs groupements hydroxy, alcoxy, amino, mono- ou di-alkylamino, alkylcarbonyle, carboxy, amido, alcoxycarbonyle, mono- ou di-alkylamino carbonyle ou un hétérocycle à 5 ou 6 atomes saturé ou insaturé ; un ou plusieurs atomes de carbone de ce radical alkyle pouvant être remplacés par un ou plusieurs hétéroatomes choisi parmi l'oxygène, l'azote et le soufre, ou par une ou plusieurs fonctions carbonyles,
le groupement R₂ représente un atome d'hydrogène ou d'halogène, un radical choisi parmi alkyle, alcoxy, alcoxy alkyle, hydroxy alcoxy ou mono- ou poly-hydroxy alkyle,
n représente un entier variant de 1 à 4.

8. Procédé de préparation du composé de formule (I) tel que défini dans les revendications 1 à 6, **caractérisé par le fait qu'**on effectue une étape de réduction d'un composé nitré correspondant au composé de formule (I) défini dans les revendications 1 à 6.

9. Utilisation, du composé de formule (I), 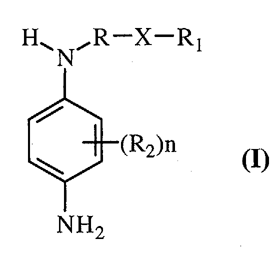 dans laquelle :
le groupement X représente un radical SO₂ ou un atome de soufre,
si X représente un radical SO₂, le groupement R représente un radical alkylène en C₂-C₁₀ linéaire ou ramifié, saturé ou insaturé, non-substitué ou substitué par un atome d'halogène ou par un ou plusieurs groupements hydroxy, alcoxy, amino, mono- ou di-alkylamino, alkylcarbonyle, carboxy, amido, alcoxycarbonyle ou mono- ou di-alkylamino carbonyle ; un ou plusieurs atomes de carbone de ce radical alkylène pouvant être remplacés par un ou plusieurs hétéroatomes choisi parmi l'oxygène, l'azote et le soufre, ou par une ou plusieurs fonctions carbonyles,
si X représente un atome de soufre, le groupement R représente un radical alkylène en C₁-C₁₀ ramifié, saturé ou insaturé, non-substitué ou substitué par un atome d'halogène ou par un ou plusieurs groupements hydroxy, alcoxy, amino, mono- ou di-alkylamino, alkylcarbonyle, carboxy, amido, alcoxycarbonyle ou mono- ou di-alkylamino carbonyle ; un ou plusieurs atomes de carbones de ce radical alkylène pouvant être remplacés par un ou plusieurs hétéroatomes choisi parmi l'oxygène, l'azote et le soufre, ou par une ou plusieurs fonctions carbonyles,
le groupement R₁ représente un radical amino, un radical -OY, dans lequel Y représente un atome d'hydrogène ou un métal, un radical aryle ou un radical alkyle en C₁-C₁₀ linéaire ou ramifié, saturé ou insaturé, non-substitué ou substitué par un atome d'halogène ou par un ou plusieurs groupements hydroxy, alcoxy, amino, mono- ou di-alkylamino, alkylcarbonyle, carboxy, amido, alcoxycarbonyle, mono- ou di-alkylamino carbonyle ou un hétérocycle à 5 ou 6 atomes saturé ou insaturé ; un ou plusieurs atomes de carbone de ce radical alkyle pouvant être remplacés par un ou plusieurs hétéroatomes choisi parmi l'oxygène, l'azote et le soufre, ou par une ou plusieurs fonctions carbonyles,
le groupement R₂ représente un atome d'hydrogène ou d'halogène, un radical choisi parmi alkyle, alcoxy, alcoxy alkyle, hydroxy alcoxy ou mono- ou poly-hydroxy alkyle,
n représente un entier variant de 1 à 4,
pour la préparation d'une composition cosmétique pour la teinture des fibres kératiniques, de préférence les fibres kératiniques telles que les cheveux
à l'exception des composés suivants :
- Acide 2-(4-amino-3-méthyl-phénylamino)-éthanesulfonique amide,
- Acide 2-(4-amino-phénylamino)-éthanesulfonique diéthylamide,
- N-[2-(4-Amino-2-méthyl-phénylamino)-éthyl]-méthanesulfonamide,
- N-[2-(4-Amino-phénylamino)-éthyl]-méthanesulfonamide

10. Composition cosmétique pour la teinture des fibres, de préférence les fibres kératiniques humaines telles que les cheveux comprenant dans un milieu approprié pour la teinture au moins un composé de formule générale (I) : 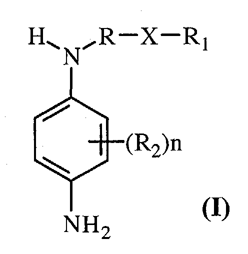 dans laquelle :
le groupement X représente un radical SO₂ ou un atome de soufre,
si X représente un radical SO₂, le groupement R représente un radical alkylène en C₂-C₁₀ linéaire ou ramifié, saturé ou insaturé, non-substitué ou substitué par un atome d'halogène ou par un ou plusieurs groupements hydroxy, alcoxy, amino, mono- ou di-alkylamino, alkylcarbonyle, carboxy, amido, alcoxycarbonyle ou mono- ou di-alkylamino carbonyle ; un ou plusieurs atomes de carbone de ce radical alkylène pouvant être remplacés par un ou plusieurs hétéroatomes choisi parmi l'oxygène, l'azote et le soufre, ou par une ou plusieurs fonctions carbonyles,
si X représente un atome de soufre, le groupement R représente un radical alkylène en C₁-C₁₀ ramifié, saturé ou insaturé, non-substitué ou substitué par un atome d'halogène ou par un ou plusieurs groupements hydroxy, alcoxy, amino, mono- ou di-alkylamino, alkylcarbonyle, carboxy, amido, alcoxycarbonyle ou mono- ou di-alkylamino carbonyle ; un ou plusieurs atomes de carbones de ce radical alkylène pouvant être remplacés par un ou plusieurs hétéroatomes choisi parmi l'oxygène, l'azote et le soufre, ou par une ou plusieurs fonctions carbonyles,
le groupement R₁ représente un radical amino, un radical -OY, dans lequel Y représente un atome d'hydrogène ou un métal, un radical aryle ou un radical alkyle en C₁-C₁₀ linéaire ou ramifié, saturé ou insaturé, non-substitué ou substitué par un atome d'halogène ou par un ou plusieurs groupements hydroxy, alcoxy, amino, mono- ou di-alkylamino, alkylcarbonyle, carboxy, amido, alcoxycarbonyle, mono- ou di-alkylamino carbonyle ou un hétérocycle à 5 ou 6 atomes saturé ou insaturé ; un ou plusieurs atomes de carbone de ce radical alkyle pouvant être remplacés par un ou plusieurs hétéroatomes choisi parmi l'oxygène, l'azote et le soufre, ou par une ou plusieurs fonctions carbonyles,
le groupement R₂ représente un atome d'hydrogène ou d'halogène, un radical choisi parmi alkyle, alcoxy, alcoxy alkyle, hydroxy alcoxy ou mono- ou poly-hydroxy alkyle,
n représente un entier variant de 1 à 4,
ou ses sels d'addition
à l'exception des composés suivants :
- Acide 2-(4-amino-3-méthyl-phénylamino)-éthanesulfonique amide,
- Acide 2-(4-amino-phénylamino)-éthanesulfonique diéthylamide,
- N-[2-(4-Amino-2-méthyl-phénylamino)-éthyl]-méthanesulfonamide,
- N-[2-(4-Amino-phénylamino)-éthyl]-méthanesulfonamide.

11. Composition selon la revendication 10, **caractérisée en ce que** le groupement R de la formule (I) représente un alkylène linéaire ou ramifié en C₂-C₅, pouvant être substitué par un radical choisi parmi -OH , -COOCH₃ et -COOCH₂CH₃, un ou plusieurs atomes de carbone de ce radical alkylène pouvant être remplacés par un ou plusieurs atomes d'oxygène, d'azote ou de soufre.

12. Composition selon la revendication 10 ou 11, **caractérisée en ce que** le groupement R₁ de la formule (I) représente un groupement alkyle, aryle, arylalkyle, hydroxyalkyle, amine secondaire ou tertiaire.

13. Composition selon l'une quelconque des revendications 10 à 12, **caractérisée en ce que** le groupement R₂ de la formule (I) représente un groupement méthyle.

14. Composition selon l'une quelconque des revendications 10 à 13, **caractérisée en ce que** le composé de formule (I) est choisi parmi les composés suivants :
- N-[2-(4-amino-phénylamino)-éthyl]-benzènesulfonamide,
- Acide 2-(4-amino-phénylamino)-éthanesulfonique (tetrahydro-furan-2-ylméthyl)-amide,
- Acide 2-(4-amino-phénylamino)-éthanesulfonique méthylamide,
- Acide 2-(4-amino-phénylamino)-éthanesulfonique (2-hydroxy-éthyl)-amide,
- N-[2-(4-Amino-phénylamino)-éthyl]-méthanesulfonamide,
- Acide 2-(4-amino-phénylamino)-éthanesulfonique diéthylamide,
- Acide 2-(4-amino-phénylamino)-éthanesulfonique amide,
- N-[2-(4-Amino-phénylamino)-éthyl]-N-méthyl-méthanesulfonamide,
- N-[4-(4-Amino-phénylamino)-butyl]-méthanesulfonamide,
- Acide 2-(4-amino-phénylamino)-éthanesulfonique bis-(2-hydroxy-éthyl)-amide,
- N-(2-Méthanesulfonyl-éthyl)-benzène-1,4-diamine,
- Acide méthanéthiosulfonique S-[2-(4-amino-phénylamino)-éthyl] ester,
- Acide sulfurique mono-[2-(4-amino-phénylamino)-éthyl] ester,
- Acide thiosulfurique S-[2-(4-amino-phénylamino)-éthyl] ester,
- Acide 2-(4-amino-phénylamino)-4-méthylsulfanyl-butyrique méthyl ester,
- 2-(4-Amino-phénylamino)-3-benzylsulfanyl-propan-1-ol,
- Acide 2-(4-amino-phénylamino)-3-benzylsulfanyl-propionique éthyl ester,
- Acide 2-(4-amino-phénylamino)-3-benzylsulfanyl-propionique méthyl ester,
- Acide 2-(4-amino-phénylamino)-4-méthylsulfanyl-butyrique éthyl ester,
- N-[2-(4-Amino-2-méthyl-phénylamino)-éthyl]-benzènesulfonamide,
- Acide 2-(4-Amino-2-méthyl-phénylamino)-éthanesulfonique (tetrahydro-furan-2-ylméthyl)-amide,
- Acide 2-(4-Amino-2-méthyl-phénylamino)-éthanesulfonique méthylamide,
- Acide 2-(4-amino-2-méthyl-phénylamino)-éthanesulfonique (2-hydroxy-éthyl)-amide,
- N-[2-(4-Amino-2-méthyl-phénylamino)-éthyl]-méthanesulfonamide,
- Acide 2-(4-amino-2-méthyl-phénylamino)-éthanesulfonique diéthylamide,
- Acide 2-(4-amino-2-méthyl-phénylamino)-éthanesulfonique amide,
- N-[2-(4-Amino-2-méthyl-phénylamino)-éthyl]-N-méthyl-méthanesulfonamide,
- N-[4-(4-Amino-2-méthyl-phénylamino)-butyl]-méthanesulfonamide,
- Acide 2-(4-amino-2-méthyl-phénylamino)-éthanesulfonique bis-(2-hydroxy-éthyl)-amide,
- N-1-(2-Méthanesulfonyl-éthyl)-2-méthyl-benzène-1,4-diamine,
- Acide méthanéthiosulfonique S-[2-(4-amino-2-méthyl-phénylamino)-éthyl] ester,
- Acide sulfurique mono-[2-(4-amino-2-méthyl-phénylamino)-éthyl] ester,
- Acide thiosulfurique S-[2-(4-amino-2-méthyl-phénylamino)-éthyl] ester,
- Acide 2-(4-amino-2-méthyl-phénylamino)-4-méthylsulfanyl-butyrique méthyl ester,
- 2-(4-Amino-2-méthyl-phénylamino)-3-benzylsulfanyl-propan-1-ol,
- Acide 2-(4-amino-2-méthyl-phénylamino)-3-benzylsulfanyl-propionique éthyl ester,
- Acide 2-(4-amino-2-méthyl-phénylamino)-3-benzylsulfanyl-propionique méthyl ester,
- Acide 2-(4-amino-2-méthyl-phénylamino)-4-méthylsulfanyl-butyrique éthyl ester,
- N-[2-(4-Amino-3-méthyl-phénylamino)-éthyl]-benzènesulfonamide,
- Acide 2-(4-amino-3-méthyl-phénylamino)-éthanesulfonique (tetrahydro-furan-2-ylméthyl)-amide,
- Acide 2-(4-amino-3-méthyl-phénylamino)-éthanesulfonique méthylamide,
- Acide 2-(4-amino-3-méthyl-phénylamino)-éthanesulfonique (2-hydroxy-éthyl)-amide,
- Acide 2-(4-amino-3-méthyl-phénylamino)-éthanesulfonique diéthylamide,
- Acide 2-(4-amino-3-méthyl-phénylamino)-éthanesulfonique amide,
- N-[2-(4-Amino-3-méthyl-phénylamino)-éthyl]-N-méthyl-méthanesulfonamide,
- N-[4-(4-Amino-3-méthyl-phénylamino)-butyl]-méthanesulfonamide
- Acide 2-(4-amino-3-méthyl-phénylamino)-éthanesulfonique bis-(2-hydroxy-éthyl)-amide,
- N-4-(2-Méthanesulfonyl-éthyl)-2-méthyl-benzène-1,4-diamine,
- Acide méthanéthiosulfonique S-[2-(4-amino-3-méthyl-phénylamino)-éthyl] ester,
- Acide sulfurique mono-[2-(4-amino-3-méthyl-phénylamino)-éthyl] ester,
- Acide thiosulfurique S-[2-(4-amino-3-méthyl-phénylamino)-éthyl] ester,
- Acide 2-(4-amino-3-méthyl-phénylamino)-4-méthylsulfanyl-butyrique méthyl ester,
- 2-(4-Amino-3-méthyl-phénylamino)-3-benzylsulfanyl-propan-1-ol,
- Acide 2-(4-amino-3-méthyl-phénylamino)-3-benzylsulfanyl-propionique éthyl ester,
- Acide 2-(4-amino-3-méthyl-phénylamino)-3-benzylsulfanyl-propionique méthyl ester,
- Acide 2-(4-amino-3-méthyl-phénylamino)-4-méthylsulfanyl-butyrique éthyl ester,
- N-[2-(4-Amino-3-méthyl-phénylamino)-éthyl]-méthanesulfonamide,
et leurs sels d'addition.

15. Composition selon l'une quelconque des revendications 10 à 14, **caractérisée en ce que** les sels d'addition avec un acide des composés de formule (I) sont choisis parmi les chlorhydrates, les bromhydrates, les sulfates, les citrates, les succinates, les tartrates, les lactates, les tosylates, les benzènesulfonates, les phosphates et les acétates.

16. Composition selon l'une quelconque des revendications 10 ou 15, **caractérisée en ce que** la concentration du composé de formule générale (I) est comprise entre 0,0001 et 20 %, de préférence entre 0,005 à 6 % en poids par rapport au poids total de la composition.

17. Composition selon l'une quelconque des revendications 10 ou 16, **caractérisée en ce que** le milieu approprié pour la teinture est constitué par de l'eau ou par un mélange d'eau et d'au moins un solvant organique choisi parmi les alcools inférieurs en C₁-C₄, ramifiés ou non; les polyols et éthers de polyols; les alcools aromatiques et leurs mélanges.

18. Composition selon l'une quelconque des revendications 10 à 17, **caractérisée en ce qu'**elle comprend au moins un adjuvant cosmétique choisi parmi les agents antioxydants, les agents de pénétration, les agents séquestrants, les parfums, les tampons, les agents dispersants, les tensioactifs, les agents de conditionnement, les agents filmogènes, les polymères, les céramides, les agents conservateurs, les agents nacrants ou opacifiants, les vitamines ou provitamines.

19. Composition selon la revendication 18, **caractérisée en ce que** la quantité en adjuvant cosmétique est comprise pour chacun d'eux entre 0,01 et 20 % en poids par rapport au poids total de la composition.

20. Composition selon l'une quelconque des revendications 10 à 19, **caractérisée en ce que** la composition comprend au moins un coupleur choisi parmi les méta-phénylènediamines, les méta-aminophénols, les méta-diphénols, les coupleurs naphtaléniques, les coupleurs hétérocycliques ainsi que leurs sels d'addition.

21. Composition selon la revendication 20, **caractérisée en ce que** la concentration du ou des coupleurs est comprise entre 0,0001 et 20 % en poids par rapport au poids total de la composition.

22. Composition selon l'une quelconque des revendications 10 à 21, **caractérisée en ce que** la composition comprend au moins une base d'oxydation additionnelle différente du composé de formule (I) choisie parmi les para-phénylènediamines, les bis-phénylalkylènediamines, les para-aminophénols, les ortho-aminophénols, les bases hétérocycliques et leurs sels d'addition.

23. Composition selon la revendication 22, **caractérisée en ce que** la concentration de la ou les bases d'oxydation est comprise entre 0,0001 et 20 % en poids par rapport au poids total de la composition.

24. Composition selon l'une quelconque des revendications 10 à 23, **caractérisée en ce qu'**elle renferme un ou plusieurs colorants directs cationiques ou naturels.

25. Procédé de teinture des fibres kératiniques et en particulier des fibres kératiniques humaines telles que les cheveux, **caractérisée par le fait qu'**on applique sur lesdites fibres au moins une composition selon l'une quelconque des revendications 10 à 24, pendant une durée suffisante pour développer la coloration désirée en présence d'un agent oxydant.

26. Composition prête à l'emploi, **caractérisée en ce qu'**elle comprend dans une composition tinctoriale telles que définie dans l'une quelconque des revendications 10 à 24 au moins un agent oxydant choisi parmi le peroxyde d'hydrogène, le peroxyde d'urée, les bromates de métaux alcalins, les persels, les peracides et les enzymes oxydases, et de préférence le peroxyde d'hydrogène.

27. Utilisation pour la teinture des fibres kératiniques et en particulier des fibres kératiniques humaines telles que les cheveux de la composition telle que définie dans l'une quelconque des revendications 10 à 24.

28. Dispositif à plusieurs compartiments dans lequel le premier compartiment contient une composition tinctoriale pour la teinture des fibres kératiniques telle que définie dans l'une quelconque des revendications 10 à 24 et un deuxième compartiment contient un agent oxydant.
